# EUROPEAN PATENT APPLICATION

(11) **EP 4 260 861 A1**
(43) Date of publication of application: **18.10.2023**
(21) Application number: 23167591.9
(22) Date of filing: 12.04.2023
(51) Int. Cl.: A61K 31/5415, A61P 35/00, G01N 33/574

(54) **PHARMACEUTICAL COMPOSITION FOR ANTI-CANCER COMPRISING NUPR1 ISOFORM A INHIBITOR**

(30) Priority: 13.04.2022 KR 20220045625
(71) Applicant: Seoul National University R & DB Foundation, Seoul 08826 (KR); Brain & Beyonds Co., Ltd., Seoul 03122 (KR)
(72) Inventor: PAEK, Sun Ha, SEOUL (KR); KIM, Yona, SEOUL (KR); YOU, Ji Hyeon, SEOUL (KR); PARK, Soon Beom, SEOUL (KR)
(74) Representative: Algemeen Octrooi- en Merkenbureau B.V.

(57) **Abstract**

The present disclosure provides a pharmaceutical composition for preventing or treating cancer comprising a NUPR1 isoform A inhibitor as an active ingredient, a composition for diagnosing or predicting prognosis of cancer comprising an agent for measuring an expression or activity level of a NUPR1 isoform A, a kit for diagnosing or predicting prognosis of cancer comprising the composition, a method for screening a substance for inhibiting cancer cell proliferation or metastasis, a method for providing information for cancer cell proliferation or metastasis, a food composition for preventing or improving cancer comprising a NUPR1 isoform A inhibitor as an active ingredient, and a feed composition for preventing or improving cancer comprising a NUPR1 isoform A inhibitor as an active ingredient.

Since a NUPR1 isoform A inhibitor according to the present disclosure has an anticancer effect, pharmaceutical compositions, food compositions, and feed compositions comprising the NUPR1 isoform A inhibitor may be usefully used for preventing, treating, or improving cancer. In addition, by measuring the expression or activity level of a NUPR1 isoform A, cancer diagnosis or prognosis may be predicted, substances for inhibiting cancer growth or metastasis may be screened, and information for cancer growth or metastasis may be provided to a subject.

## Description

### [Technical Field]

The present disclosure relates to a pharmaceutical composition for preventing or treating cancer comprising a NUPR1 isoform A inhibitor as an active ingredient, an anticancer adjuvant for anticancer drug-resistant cancer, an anticancer drug resistance or sensitivity enhancer comprising a NUPR1 isoform A inhibitor as an active ingredient, a composition for diagnosing or predicting prognosis of cancer comprising an agent for measuring an expression or activity level of a NUPR1 isoform A, a kit for diagnosing or predicting prognosis of cancer comprising the composition, a method for screening a substance for inhibiting cancer cell proliferation or metastasis, a method for providing information for cancer cell proliferation or metastasis, a food composition for preventing or improving cancer comprising a NUPR1 isoform A inhibitor as an active ingredient, and a feed composition for preventing or improving cancer comprising a NUPR1 isoform A inhibitor as an active ingredient.

### [Background Art]

Despite the development of modern medicine, cancer is still one of the most incurable diseases among human diseases. Cancer is a tissue made up of abnormal cells that develop in one or more tissues and spread to other tissues. Currently, 100 or more types of cancer are known.

It is known that 90-95% of the main causes of cancer are genetic mutations caused by environmental factors, and most of these factors include smoking, obesity, radiation, UV, stress, and environmental pollution, etc.

Currently, cancer treatment methods may use chemotherapy using drugs such as antimetabolites, alkylating agents, topoisomerase inhibitors, and monoclonal antibodies, simultaneously with radiation therapy. However, these conventional methods may differ for each patient, but generally have side effects such as hair loss, inhibition of bone marrow function, drug resistance, and organ damage. Numerous efforts are being made all over the world to overcome these side effects and conquer cancer, and as one part of these efforts, studies to prevent the expression of proteins related to the development or proliferation of cancer have been actively conducted.

On the other hand, NUPR1 (Nuclear protein 1) is a small chromatin protein that is known to respond to various stress stimuli and is known to be involved in cell cycle, DNA damage response, apoptosis, autophagy, chromatin remodeling, etc., as a transcriptional regulator.

The mRNA transcript of NUPR1 is 600 bp nucleotides in length and forms two isomers, NUPR1A and NUPR1B, through alternative splicing, and differences in function and expression patterns between the two isomers have not yet been reported, and most NUPR1 relevant studies have focused on NUPR1 isoform B.

Under this background, the present inventors have made intensive studies on the prevention or treatment of cancer by inhibiting the expression of proteins related to the development or proliferation of cancer, and have completed the present disclosure by confirming that various cancers may be treated by inhibiting the expression of a NUPR1 isoform A.

### [Related Art Document]

### [Patent Document]

(Patent Document 0001) Korean Patent Laid-Open Publication No. 10-2015-0003946

### [Disclosure]

### [Technical Problem]

The object of present disclosure is to solve the above problems and other problems related thereto.

An exemplary object of the present disclosure is to provide a pharmaceutical composition for preventing or treating cancer, comprising a NUPR1 isoform A inhibitor as an active ingredient.

An exemplary object of the present disclosure is to provide an anticancer adjuvant for a cancer resistant to an anticancer drug, comprising a NUPR1 isoform A inhibitor as an active ingredient.

An exemplary object of the present disclosure is to provide an agent for treating anticancer drug resistance or for enhancing sensitivity to treat anticancer drug resistance or increase the effect of enhancing anticancer drug sensitivity, comprising a NUPR1 isoform A inhibitor as an active ingredient.

Another exemplary object of the present disclosure is to provide a composition for diagnosing or predicting prognosis of cancer, comprising an agent for measuring an expression or activity level of a NUPR1 isoform A.

Another exemplary object of the present disclosure is to provide a kit for diagnosing or predicting prognosis of cancer, comprising the composition.

Another exemplary object of the present disclosure is to provide a method for screening a substance for inhibiting a cancer cell proliferation or metastasis, including:
treating a candidate substance to a cancer cell line expressing a NUPR1 isoform A; and
measuring an expression level of the NUPR1 isoform A in the cancer cell line.

Another exemplary object of the present disclosure is to provide a method for providing an information for cancer cell proliferation and metastasis, including:
measuring an expression level of mRNA or protein of a NUPR1 isoform A in a sample derived from a subject; and
determining the subject as an individual at risk of cancer cell proliferation or metastasis wherein the expression level of mRNA or protein of the NUPR1 isoform A in the subject is increased than a control.

An exemplary object of the present disclosure is to provide a food composition for preventing or improving cancer comprising a NUPR1 isoform A inhibitor as an active ingredient.

An exemplary object of the present disclosure is to provide a feed composition for preventing or improving cancer comprising a NUPR1 isoform A inhibitor as an active ingredient.

The technical challenge to be achieved according to the technical idea of the present disclosure disclosed in the specification is not limited to the challenge to solve the problems mentioned above, and another challenge not mentioned above will be clearly understood by those skilled in the art from the following description.

### [Technical Solution]

This will be explained in details as follows. Meanwhile, each of description and embodiment disclosed in the present application may also be applied to each of other description and embodiment. That is, all combinations of various elements disclosed in the present application fall within the scope of the present application. In addition, the scope of the present application is not to be construed as being limited by the specific descriptions below.

As one aspect for achieving the above object, the present disclosure provides a composition comprising a NUPR1 isoform A inhibitor as an active ingredient. The composition may be a pharmaceutical composition or a food composition. In an aspect the composition is for use in the prevention or treatment of cancer.

Nuclear protein 1 (NUPR1) is a small chromatin protein that is known to respond to various stress stimuli and is known to be involved in cell cycle, DNA damage response, apoptosis, autophagy and chromatin remodeling as a transcriptional regulator.

The mRNA transcript of NUPR1 forms two isomers, a NUPR1 isoform A and NUPR1 isoform B, and differences in function and expression patterns between the two isomers have not yet been reported, and most NUPR1 relevant studies have focused on NUPR1 isoform B.

The NUPR1 isoform A is one of the NUPR1 isomers and is also referred to as "NUPR1 variant 1 (variant 1, v.1.)" or "NUPR1α" (NCBI Reference Sequence ID: NP_001035948.1 and NM_001042483.1).

The "NUPR1 isoform A inhibitor" as used herein collectively refers to substances that reduce the expression or activity of a NUPR1 isoform A. An example thereof may include any substance that reduces the expression or activity of a NUPR1 isoform A by reducing the expression of a NUPR1 isoform A at the transcriptional level or interfering with its activity through the manner of acting directly on a NUPR1 isoform A or indirectly acting on its ligand, etc. As substances that inhibits the expression of a NUPR1 isoform A, compounds, nucleic acids, peptides, viruses, or vectors containing the nucleic acid, antibodies, proteins, various ligands, aptamers, etc., capable of targeting a NUPR1 isoform A and inhibiting the expression or activity of the NUPR1 isoform A, may be used without limitation in the form.

In one embodiment of the present disclosure, the NUPR1 isoform A inhibitor is 10-(4-(4-(pyrrolidin-1-yl)piperidin-1-yl)butyl)-2-trifluoromethyl)-10H-phenothiazine represented by Formula 1 or a derivative thereof:

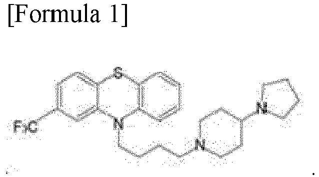

In one embodiment of the present disclosure, the NUPR1 isoform A inhibitor functions to inhibit activity or expression by binding to or indirectly acting on the NUPR1 isoform A.

In one embodiment of the present disclosure, the NUPR1 isoform A inhibitor may be any one selected from the group consisting of 10-(4-(4-phenylpiperazin-1-yl)butyl)-2-(trifluoromethyl)-10H-phenothiazine; 10-(4-(4-(2-fluorophenyl)piperazin-1-yl)butyl)-2-(trifluoromethyl)-10H-phenothiazine; 10-(3-(4-phenylpiperazin-1-yl)propyl)-2-(trifluoromethyl)-10H-phenothiazine; 10-(3-(4-(4-chlorobenzyl)piperazin-1-yl)propyl)-2-(trifluoromethyl)-10H-phenothiazine; 10-(3-(4-(4-) methoxyphenyl)piperazin-1-yl)propyl)-2-(trifluoromethyl)-10H-phenothiazine; 10-(3-(4-(4-nitrophenyl)piperazin-1-yl)propyl)-2-(trifluoromethyl)-10H-phenothiazine; 10-(3-(4-(3-chlorobenzyl)piperazin-1-yl)propyl)-2-(trifluoromethyl)-10H-phenothiazine; 10-(3-(4-(4-chlorophenyl)piperazin-1-yl)propyl)-2-(trifluoromethyl)-10-phenothiazine; 10-(3-(4-(4-fluorobenzyl)piperazin-1-yl)propyl)-2-(trifluoromethyl)-10H-phenothiazine; 10-(3-(4-(2-chlorophenyl)piperazin-1-yl)propyl)-2-(trifluoromethyl)-10H-phenothiazine; 10-(3-(4-p-tolylpiperazin-1-yl)propyl)-2-(trifluoromethyl)-10H-phenothiazine; 10-(3-(4-phenethylpiperazin-1-yl)propyl)-2-(trifluoromethyl)-10H-phenothiazine; 10-(4-(4-(4-nitrophenyl)piperazin-1-yl)butyl)-2-(trifluoromethyl)-10H-phenothiazine; 10-(4-(4-(4-chlorophenyl)piperazin-1-yl)butyl)-2-(trifluoromethyl)-10H-phenothiazine; 2-chloro-10-(4-(4-phenylpiperazin-1-yl)butyl)-10H-phenothiazine; 2-chloro-10-(4-(4-(4-nitrophenyl)piperazin-1-yl)butyl)-10H-phenothiazine; 2-chloro-10-(4-(4-(4-methoxyphenyl)piperazin-1-yl)butyl)-10H-phenothiazine; 2-chloro-10-(4-(4-(4-chlorophenyl)piperazin-1-yl)butyl)-10H-phenothiazine; 10-(4-(4-(4-methoxyphenyl)piperazin-1-yl)butyl)-2-(trifluoromethyl)-10H-phenothiazine; 2-chloro-10-(3-(4-(4-methoxyphenyl)piperazin-1-yl)propyl)-10H-phenothiazine; 2-chloro-10-(3-(4-phenylpiperazin-1-yl)propyl)-10H-phenothiazine; 2-chloro-10-(3-(4-(4-nitrophenyl)piperazin-1-yl)propyl)-10H-phenothiazine; 2-chloro-10-(3-(4-(4-chlorophenyl)piperazin-1-yl)propyl)-10H-phenothiazine; 10-(4-(4-phenylpiperidin-1-yl)butyl)-2-(trifluoromethyl)-10H-phenothiazine; 10-(4-(4-(4-methoxyphenyl)piperazin-1-yl)butyl)-2-(methylthio)-10H-phenothiazine; 2-(methylthio)-10-(4-(4-phenylpiperazin-1-yl)butyl)-10H-phenothiazine; 10-(3-(4-phenylpiperidin-1-yl)propyl)-2-(trifluoromethyl)-10H-phenothiazine; 2-(methylthio)-10-(4-(4-(4-nitrophenyl)piperazin-1-yl)butyl)-10H-phenothiazine; 10-(4-(4-(4-chlorophenyl)piperazin-1-yl)butyl)-2-(methylthio)-10H-phenothiazine; 2-(methylthio)-10-(3-(4-phenylpiperazin-1-yl)propyl)-10H-phenothiazine; 10-(3-(4-(4-chlorophenyl)piperazin-1-yl)propyl)-2-(methylthio)-10H-phenothiazine; 2-(methylthio)-10-(3-(4-(4-nitrophenyl)piperazin-1-yl)propyl)-10H-phenothiazine; 10-(3-(4-(4-methoxyphenyl)piperazin-1-yl)propyl)-2-(methylthio)-10H-phenothiazine; 2-chloro-10-(4-(4-phenylpiperidin-1-yl)butyl)-10H-phenothiazine; 2-chloro-10-(3-(4-phenylpiperidin-1-yl)propyl)-10H-phenothiazine; 2-(methylthio)-10-(3-(4-phenylpiperidin-1-yl)propyl)-10H-phenothiazine; 2-(methylthio)-10-(4-(4-phenylpiperidin-1-yl)butyl)-10H-phenothiazine; 10-(3-(1H-benzo[d]imidazol-1-yl)propyl)-2-(trifluoromethyl)-10H-phenothiazine; 2-(trifluoromethyl)-10-(3-(2,5,6-trimethyl-1H-benzo[d]imidazol-1-yl)propyl)-10H-phenothiazine; 2-(1-(4-(2-(trifluoromethyl)-10H-phenothiazine;-10-yl)butyl)piperidin-4-yl)ethanol; 4-(1-(4-(2-(trifluoromethyl)-10H-phenothiazine;-10-yl)butyl)piperidin-4-yl)morpholine; 10-(4-(4-(pyrrolidin-1-yl)piperidin-1-yl)butyl)-2-(trifluoromethyl)-10H-phenothiazine; 10-(4-(1,4'-bipiperidin-1'-yl)butyl)-2-(trifluoromethyl)-10H-phenothiazine; 4-(1-(4-(2-chloro-10H-phenothiazine;-10-yl)butyl)piperidin-4-yl)morpholine; 4-(1-(4-(2-(methylthio)-10H-phenothiazine;-10-yl)butyl)piperidin-4-yl)morpholine; 4-(1-(3-(2-(trifluoromethyl)-10H-phenothiazine;-10-yl)propyl)piperidin-4-yl)morpholine; 10-(4-(4,4-difluoropiperidin-1-yl)butyl)-2-(trifluoromethyl)-10H-phenothiazine; 10-(3-(4-(pyrrolidin-1-yl)piperidin-1-yl)propyl)-2-(trifluoromethyl)-10H-phenothiazine; 4-(1-(3-(2-chloro-10H-phenothiazine;-10-yl)propyl)piperidin-4-yl)morpholine; 2-chloro-10-(4-(4-(pyrrolidin-1-yl)piperidin-1-yl)butyl)-10H-phenothiazine; 2-chloro-10-(3-(4-(pyrrolidin-1-yl)piperidin-1-yl)propyl)-10H-phenothiazine; 2-(methylthio)-10-(4-(4-(pyrrolidin-1-yl)piperidin-1-yl)butyl)-10H-phenothiazine; 10-(3 -(1,4'-bipiperidin-1'-yl)propyl)-2-(trifluoromethyl)-10H-phenothiazine; 4-(1-(3-(2-(methylthio)-10H-phenothiazine;-10-yl)propyl)piperidin-4-yl)morpholine; 2-(methylthio)-10-(3-(4-(pyrrolidin-1-yl)piperidin-1-yl)propyl)-10H-phenothiazine; 10-(4-(4,4-dimethylpiperidin-1-yl)butyl)-2-(trifluoromethyl)-10H-phenothiazine; 4-(1-(4-(10H-phenothiazine;-10-yl)butyl)piperidin-4-yl)morpholine; 10-(4-(4-(pyrrolidin-1-yl)p peridin-1-yl)butyl)-10H-phenothiazine; 10-(4-(1,4'-bipiperidin-1'-yl)butyl)-10H-phenothiazine; 2-methoxy-10-(4-(4-(pyrrolidin-1-yl)piperidin-1-yl)butyl)-10H-phenothiazine; 10-(4-(1,4'-bipiperidin-1'-yl)butyl)-2-methoxy-10H-phenothiazine; 4-(1-(4-(2-methoxy-10H-phenothiazine;-10-yl)butyl)piperidin-4-yl)morpholine; 10-(3-(4-(pyrrolidin-1-yl)piperidin-1-yl)propyl)-10H-phenothiazine; 10-(3-(1,4'-bipiperidin-1'-yl)propyl)-10H-phenothiazine; 4-(1-(3 -(10H-phenothiazine;10-yl)propyl)piperidin-4-yl)morpholine; 10-(3-(1,4'-bipiperidin-1'-yl)propyl)-2-methoxy-10H-phenothiazine; 4-(1-(3-(2-methoxy-10H-phenothiazine;-10-yl)propyl)piperidin-4-yl)morpholine; 2-methoxy-10-(3-(4-(pyrrolidin-1-yl)piperidin-1-yl)propyl)-10H-phenothiazine; 10-(3-(1,4'-bipiperidin-1'-yl)propyl)-2-chloro-10H-phenothiazine; 10-(4-(1,4'-bipiperidin-1'-yl)butyl)-2-chloro-10H-phenothiazine; 10-(3 -(1,4'-bipiperidin-1'-yl)propyl)-2-(methylthio)-10H-phenothiazine; 10-(4-(1,4'-bipiperidin-1'-yl)butyl)-2-(methylthio)-10H-phenothiazine; 4-(1-(4-(2-fluoro-10H-phenothiazine;-10-yl)butyl)piperidin-4-yl)morpholine; 2-fluoro-10-(3-(4-methylpiperidin-1-yl)propyl)-10H-phenothiazine; 4-(1-(3-(2-fluoro-10H-phenothiazine;-10-yl)butyl)piperidin-4-yl)morpholine; 2-fluoro-10-(4-(4-methylpiperidin-1-yl)butyl)-10H-phenothiazine; 2-fluoro-10-(4-(4-(pyrrolidin-1-yl)piperidin-1-yl)butyl)-10H-phenothiazine; 10-(4-(1,4 '-bipiperidin-1 '-yl)butyl)-2-fluoro-10H-phenothiazine; 4-(1-(3-(2-fluoro-10H-phenothiazine;-10-yl)propyl)piperidin-4-yl)morpholine; 10-(3-(1,4'-bipiperidin-1'-yl)propyl)-2-fluoro-10H-phenothiazine; 2-fluoro-10-(3-(4-(pyrrolidin-1-yl)piperidin-1-yl)propyl)-10H-phenothiazine; 3-methyl-10-(4-(4-(pyrrolidin-1-yl)piperidin-1-yl)butyl)-10H-phenothiazine; 10-(4-(1,4'-bipiperidin-1'-yl)butyl)-3-methyl-10H-phenothiazine; 4-(1-(4-(3-methyl-10H-phenothiazine;-10-yl)butyl)piperidin-4-yl)morpholine; 4-(1-(3-(3-methyl-10H-phenothiazine;-10-yl)propyl)piperidin-4-yl)morpholine; 10-(3-(1,4'-bipiperidin-1'-yl)propyl)-3-methyl-10H-phenothiazine; 3-methyl-10-(3-(4-(pyrrolidin-1-yl)piperidin-1-yl)propyl)-10H-phenothiazine; 10-(4-(4-morpholinopiperidin-1-yl)butyl)-10H-phenothiazine;-3-carbonitrile; 10-(4-(4-(pyrrolidin-1-yl)piperidin-1-yl)butyl)-10H-phenothiazine;-3-carbonitrile; 10-(4-(1,4'-bipiperidin-1'-yl)butyl)-10H-phenothiazine;-3-carbonitrile; 2-methyl-10-(4-(4-(pyrroli din-1-yl)pip eridin-1-yl)butyl)-10H-phenothiazine; and 10-(4-(1,4'-bipiperidin-1'-yl)butyl)-2-methyl-10H-phenothiazine.

In one embodiment of the present disclosure, the NUPR1 isoform A inhibitor may be ZZW-115 represented by Formula 2 below:

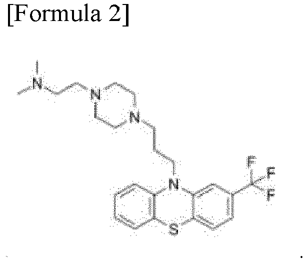

In the present disclosure, the composition may inhibit proliferation or metastasis of cancer cells by suppressing NUPR1 isoform A gene expression or NUPR1 isoform A protein expression.

In one embodiment of the present disclosure, the cancer may be, but is not limited to, at least one selected from the group consisting of brain tumor, lung cancer, breast cancer, stomach cancer, large intestine cancer, kidney cancer, skin cancer, pancreatic cancer, liver cancer, bladder cancer, colon cancer, thyroid cancer, oral cancer, prostate cancer, rectal cancer, non-small cell lung cancer, labrum cancer, anal cancer, vulvar cancer, oropharyngeal cancer, nasopharyngeal cancer, urethral cancer, small intestine cancer, biliary tract cancer, ovarian cancer, laryngeal cancer, and esophageal cancer.

The term "prevention" as used herein refers to any action that inhibits or delays cancer through administration of the pharmaceutical composition of the present disclosure, and the term "treatment" as used herein refers to any action by which the developed cancer is ameliorated or mitigated or altered in a beneficial way with administering the pharmaceutical composition of the present disclosure. The term "improvement" as used herein refers to all actions by which the cancer is ameliorated with administrating the composition.

As one aspect for achieving said object, the present disclosure provides an anticancer adjuvant for anticancer drug-resistant cancer, comprising a NUPR1 isoform A inhibitor as an active ingredient.

As one aspect for achieving said object, the present disclosure provides a pharmaceutical composition for treating anticancer drug resistance or enhancing anticancer drug sensitivity, comprising a NUPR1 isoform A inhibitor as an active ingredient.

In the present disclosure, a NUPR1 isoform A inhibitor may comprise an agent that reduces an activity or expression level of the NUPR1 isoform A protein or an agent that reduces the expression level of a gene encoding the protein, as an active ingredient. The agent for reducing the activity or expression level of the protein of the present disclosure may comprise, but is not limited to, any one or more selected from the group consisting of a compound, a peptide, an aptamer, an antibody, and a natural product that specifically bind to the protein or a part thereof, and corresponds to a means for deriving an effect of inhibiting its activity or expression by directly or indirectly acting on the target NUPR1 isoform A protein, and may comprise anything that may be easily derived by a technique known as a method commonly used in the art, without being limited thereto.

The "aptamer" as used herein is a single-stranded nucleic acid (DNA, RNA, or modified nucleic acid) that has a stable tertiary structure in itself and may bind to a target molecule with high affinity and specificity. Since the first development of aptamer discovery technology called Systematic Evolution of Ligands by EXponential Enrichment (SELEX) (Ellington, AD and Szostak, JW., Nature 346:818-822, 1990), many aptamers capable of binding to various target molecules ranging from low-molecular-weight organic substances to peptides and membrane proteins have been continuously discovered. Aptamers are comparable to single antibodies due to their inherent high affinity (usually at the pM level) and specificity of being able to bind to target molecules, and in particular, have high potential as alternative antibodies to the extent that they are called "chemical antibodies."

The "antibody" as used herein, for example, as all of being manufactured through the protein injection or commercially purchased, may be used. In addition, the antibody comprises polyclonal antibodies, monoclonal antibodies, fragments capable of binding to an epitope, etc.

Here, the polyclonal antibody may be produced, for example, by a conventional method of injecting the protein into an animal and collecting blood from the animal to obtain antibody-containing serum. Such polyclonal antibodies may be purified by any method known in the art, and may be made from a host of any animal species, such as goat, rabbit, sheep, monkey, horse, pig, cow, dog, etc.

In addition, the monoclonal antibody may be produced by using any technique that provides for the production of antibody molecules, for example, through the cultivation of continuous cell lines. Such technologies comprise, but are not limited to, hybridoma technology, human B-cell line hybridoma technology, and EBV-hybridoma technology.

In one embodiment, an antibody produced with epitope which is all or part of the amino acid sequence for a NUPR1 isoform A (SEQ ID NO: 1), for example, a sequence having 90% or more identity to the amino acid sequence of SEQ ID NO: 2 (GPLIMPMPTSPLTPALVT), is also comprised in the antibody scope of the present disclosure.

In addition, antibody fragments containing specific binding sites for the above proteins may be produced. For example, F(ab')2 fragments, but are not limited to, may be produced by pepsin digestion of antibody molecules, and Fab fragments may be produced by reducing disulfide bridges of F(ab')2 fragments. Alternatively, monoclonal Fab fragments having the desired specificity may be quickly and conveniently identified by narrowing the Fab expression library.

In the present disclosure, the antibody may be bound to a solid substrate to facilitate subsequent steps such as washing or separation of complexes, etc. Solid substrates include, for example, synthetic resins, nitrocellulose, glass substrates, metal substrates, glass fibers, microspheres, and microbeads, etc. In addition, the synthetic resin includes, for example, polyester, polyvinyl chloride, polystyrene, polypropylene, PVDF, and nylon. etc.

In the present disclosure, the agent that reduces the activity or expression level of the protein may be one that specifically binds to the amino acid sequence represented by SEQ ID NO: 1 of the NUPR1 isoform A protein, and preferably, the composition of the present disclosure may comprise antibody specific to the NUPR1 isoform A protein, wherein the antibody may specifically bind to the amino acid sequence represented by SEQ ID NO: 1, but the present disclosure is not limited thereto.

The agent for reducing the expression level of the gene encoding the protein of the present disclosure may comprise, but is not limited to, any one or more selected from the group consisting of a gene encoding the protein, preferably an antisense nucleotide that binds to be complementary to the gene or a part thereof, short interfering RNA (siRNA), short hairpin RNA, and ribozyme (ribozyme). In addition, the agent corresponds to a means for deriving an effect of inhibiting its expression by directly or indirectly acting on the gene encoding the target NUPR1 isoform A protein, and may include all without being limited thereto, as long as it may be easily derived by a known technique as a method commonly used in the art.

The "antisense nucleotide" as used herein, as defined in Watson-Crick base pairing, binds (hybridizes) to a complementary nucleotide sequence of DNA, immature-mRNA or mature mRNA to interfere with the flow of genetic information from DNA to protein. The nature of antisense nucleotides specific for target sequences makes them exceptionally multifunctional. Since antisense nucleotides are long chains of monomeric units, they may be easily synthesized against the target RNA sequence. Recently, many studies have demonstrated the usefulness of antisense nucleotides as a biochemical means to study target proteins. Since many recent advances have been made in the field of oligonucleotide chemistry and the synthesis of nucleotides that exhibit improved cell line adsorption, target binding affinity, and nuclease resistance, the use of antisense nucleotides may be considered as a new type of inhibitor.

The "shRNA" and "siRNA" as used herein are nucleic acid molecules capable of mediating RNA interference or gene silencing, and are used in an efficient gene knockdown method or gene therapy method because they may inhibit the expression of a target gene. shRNA has a hairpin structure formed by binding between complementary sequences within a single-stranded oligonucleotide, and *in vivo,* the shRNA is cleaved by dicer to form small RNA fragments of 21 to 25 nucleotides in size, which is a double-stranded oligonucleotide, and may specifically bind to mRNA with a complementary sequence and inhibit its expression. In addition, siRNA refers to a small double-stranded RNA fragment with 21 to 25 nucleotides in size that induces RNA interference (RNAi) by modifying target mRNA with double-stranded RNA (dsRNA).

In the present disclosure, whether any means of the shRNA and siRNA may be used, can be determined by a person skilled in the art, and a similar expression reduction effect may be expected if the mRNA sequences they target are the same. According to one embodiment of the present disclosure, a phenomenon of RNA interference (RNAi) is induced by specifically acting on the gene encoding the NUPR1 isoform A protein to cleave the gene (e.g., mRNA molecule) of the NUPR1 isoform A and the expression may be inhibited. siRNA may be synthesized chemically or enzymatically. The method for producing siRNA is not particularly limited, and methods known in the art may be used. Examples include, but are not limited to, a method for chemically synthesizing siRNA directly, a method for synthesizing siRNA using *in vitro* transcription, a method for cleaving long double-stranded RNA synthesized by *in vitro* transcription using an enzyme, an expression method through intracellular delivery of shRNA expression plasmids or viral vectors, and an expression method through intracellular delivery of polymerase chain reaction (PCR)-induced siRNA expression cassettes, etc.

The "ribozyme" as used herein refers to an RNA molecule having a catalytic activity. Ribozymes with various activities are known, the ribozymes of the NUPR1 isoform A gene comprise known or artificially produced ribozymes, and optionally ribozymes having target-specific RNA cleavage activity may be produced by known standard techniques.

In an example of the present disclosure, an agent for reducing the expression level of the gene encoding the NUPR1 isoform A protein may be, but is not limited to, siRNA consisting of a based sequence represented by at least one combination selected from the group consisting of SEQ ID NO: 3 and SEQ ID NO: 4; SEQ ID NO: 5 and SEQ ID NO: 6; SEQ ID NO: 7 and SEQ ID NO: 8; SEQ ID NO: 9 and SEQ ID NO: 10; or SEQ ID NO: 11 and SEQ ID NO: 12:
NUPR1a #1 F: GGCCUCUCAUCAUGCCUAUUU(SEQ ID NO: 3)
NUPR1a #1 R: AUAGGCAUGAUGAGAGGCCUU(SEQ ID NO: 4)
NUPR1a #2 F: GCCUAUGCCCACUUCACCUUU(SEQ ID NO: 5)
NUPR1a #2 R: AGGUGAAGUGGGCAUAGGCUU(SEQ ID NO: 6)
NUPR1a #3 F: GCCCACUUCACCUCUGACUUU(SEQ ID NO: 7)
NUPR1a #3 R: AGUCAGAGGUGAAGUGGGCUU(SEQ ID NO: 8)
NUPR1a #4 F: CACCUCUGACUCCUGCCUUUU(SEQ ID NO: 9)
NUPR1a #4 R: AAGGCAGGAGUCAGAGGUGUU(SEQ ID NO: 10)
NUPR1a #5 F: GACUCCUGCCUUGGUUACAUU(SEQ ID NO: 11)
NUPR1a #5 R: UGUAACCAAGGCAGGAGUCUU(SEQ ID NO: 12).

The composition of the present disclosure may further comprise a NUPR1 isoform A inhibitor to treat anticancer drug resistance or increase the effect of enhancing anticancer drug sensitivity.

In addition, the composition of the present disclosure may further comprise anticancer drug and said anticancer drug may be, without limitation, a drug comprising one or more selected from the group consisting of nitrogen mustard, imatinib, oxaliplatin, rituximab, erlotinib, neratinib, lapatinib, gefitinib, vandetanib, nitrotinib, semasanib, bosutinib, akcitinib, cediranib, restaurtinib, trastuzumab, gefitinib, bortezomib, sunitinib, carboplatin, bevacizumab, cisplatin, cetuximab, viscumalbum, asparaginase, tretinoin, hydroxycarbamide, dasatinib, estramustine, gemtuzumab ozogamycin, ibritumomabtucetan, heptaplatin, methylaminolevulinic acid, amsacrine, alemtuzumab, procarbazine, alprostadil, holmium nitrate chitosan, gemcitabine, doxyfluridine, pemetrexed, tegafur, capecitabine, gimeracin, oteracil, azacitidine, methotrexate, uracil, cytarabine, 5-fluorouracil, fludagabin, enocitabine, flutamide, decitabine, mercaptopurine, thioguanine, cladribine, carmophor, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleromycin, daunorubicin, dactinomycin, pyrarubicin, aclarubicin, pepromycin, temsirolimus, temozolomide, busulfan, ifosfamide, cyclophosphamide, melpharan, altrethamine, dacarbazine, thiotepa, nimustine, chlorambucil, mitoractol, leucovorin, tretonin, exmestane, aminoglutesimide, anagrelide, navelbin, padrazole, tamoxifen, toremifen, testoractone, anastrozole, letrozole, borozole, bicalutamide, lomustine, and carmustine.

The "Anti-cancer drug resistance" as used herein refers to the decrease in the effectiveness of the drug when the anti-cancer drug is used repeatedly in a quantitative manner, and refers to a condition in which the amount of use or the frequency of use needs to be increased in order to obtain the same effect previously experienced by patients with anti-cancer drug resistance, or the same effect may not be obtained even if the substance is administered in the same amount as before.

The "treatment of resistance" as used herein refers to recovering the condition in which the effectiveness of the drug is decreased when the anti-cancer drug is used repeatedly in a quantitative manner, or the amount of use or the frequency of use needs to be increased in order to obtain the same effect previously experienced by patients with anti-cancer drug resistance, or the same effect may not be obtained even if the substance is administered in the same amount as before. More specifically, it refers to the action of making a condition in which the same anticancer effect may be obtained even when the anticancer drug is applied less frequently or at a lower dose, or the same effect may be obtained even if the same dose or a lower dose is administered, by returning the anticancer drug to the state before the occurrence of anticancer drug resistance.

In the pharmaceutical composition for treating anticancer drug resistance and the composition for enhancing anticancer drug sensitivity of the present disclosure, descriptions of a NUPR1 isoform A protein, various anticancer drug, etc. are the same as those described in the foregoing diagnostic composition.

As used herein, the term "pharmaceutical composition" means a composition to be prepared for the purpose of preventing or treating a disease, and may be formulated and used in various forms according to conventional methods, respectively.

The pharmaceutical composition of the present disclosure may further comprise a pharmaceutically acceptable carrier, excipient or diluent according to conventional methods. Pharmaceutically acceptable carriers are well known in the art depending on the route of administration or formulation, and specific reference may be made to the pharmacopoeia of each country including the 'Korean Pharmacopoeia'. Examples of carriers, excipients and diluents that may be included in the composition of the present disclosure may include, but are not limited to, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil. In addition, examples of carriers, excipients, and diluents that may be included in the composition of the present disclosure may include, but are not limited to, non-natural carriers.

The pharmaceutical composition of the present disclosure may be formulated and used, for example, in the form of oral dosage such as powders, granules, tablets, capsules, suspensions, emulsions, syrups, aerosols, etc., external preparations, suppositories, or sterile injectable solutions, according to conventional methods. In detail, when formulated, it may be produced using diluents or excipients such as fillers, extenders, binders, wetting agents, disintegrants, and surfactants, etc., which are conventionally used. Solid formulation for oral administration comprises tablets, pills, powders, granules, capsules, etc., and the solid formulation may be produced by mixing at least one excipient, for example, starch, calcium carbonate, sucrose, lactose, gelatin, etc. with the compounds. Also, in addition to simple excipients, lubricants such as magnesium stearate and talc may be used. A liquid formulation for oral administration may comprise suspensions, oral liquids, emulsions, syrups, etc., and comprise various excipients, for example, a wetting agent, a sweetener, an aromatic, a preservative, etc., in addition to water and liquid paraffin which are a simple diluent commonly used. Examples of Formulation for parenteral administration includes sterilized aqueous solutions, non-aqueous solutions, suspensions, emulsions, lyophilized formulations, and suppositories. As the non-aqueous solvent and suspending agent, propylene glycol, polyethylene glycol, vegetable oil such as olive oil, and injectable ester such as ethyl oleate may be used. As a base for suppositories, witepsol, macrogol, tween 61, cacao butter, Laurin, glycerogelatin, etc. may be used. The specific formulation of the pharmaceutical composition is known in the art, and reference may be made to, for example, Remington's Pharmaceutical Sciences (19th ed., 1995). Said document is considered as part of this specification.

The pharmaceutical composition of the present disclosure is administered in a pharmaceutically effective amount. The pharmacologically effective amount means an amount that is sufficient to treat a disease at a reasonable benefit/risk ratio applicable to medical treatment and does not cause side effects, and the level of the effective amount may be determined according to the health state of the patient, kind of the disease, severity, drug activity, drug sensitivity, administration method, administration time, route of administration and excretion rate, duration of treatment, factors including drugs used in combination or co-administration, and other factors well known in the medical field. The dosage and frequency do not limit the scope of the present disclosure in any way.

The pharmaceutical composition of the present disclosure may be administered to mammals such as mice, dogs, cats, cows, horses, pigs, and humans through various routes, and may be preferably administered to a human. The routes of administration of the pharmaceutical composition of the present disclosure includes, but are not limited to, oral, intravenous, intramuscular, intraarterial, intramedullary, intrathecal, intracardiac, transdermal, subcutaneous, intraperitoneal, intranasal, intestinal, topical, sublingual or rectal, intracarcinoma administration.

The "parenteral" as used herein includes subcutaneous, intradermal, intravenous, intramuscular, intraarticular, intrasynovial, intrasternal, intrathecal, intralesional and intracranial injection or infusion techniques. Preferably, the pharmaceutical composition of the present disclosure may also be administered in the form of a suppository for rectal administration, but the present disclosure is not limited thereto.

The composition according to the present disclosure may have a preventive or therapeutic effect on cancer.

As used herein, the phrase "comprising as an active ingredient" means that the pharmaceutical composition comprises an effective amount capable of exhibiting an anticancer effect.

As another aspect for achieving the above object, the present disclosure provides a use of a NUPR1 isoform A for treatment and/or prevention and/or diagnosis of cancer.

As another aspect for achieving the above object, the present disclosure provides a biomarker composition for diagnosing or predicting prognosis of cancer, comprising a NUPR1 isoform A.

The "diagnosis" as used herein means confirming the presence or character of a pathological condition. For purposes of the present disclosure, diagnosis includes confirming whether a cancer has occurred. The "prognosis" as used herein means prediction of disease progression and recovery, and refers to prospective or preliminary evaluation. For the purpose of the present disclosure, prognosis includes prediction of treatment success, survival, recurrence, metastasis, etc. in relevant subject after treatment of a cancer patient, and preferably means survival prognosis.

The "biomarker" as used herein is a substance capable of diagnosing or predicting prognosis of cancer, and may diagnose cancer by distinguishing whether or not cancer has occurred in a biological sample, and may be used as a prognostic factor for individuals with poor prognosis, and includes organic biomolecule, such as polypeptides, nucleic acids (e.g., mRNA, etc.), lipids, glycolipids, glycoproteins, sugars (monosaccharides, disaccharides, oligosaccharides, etc.) etc., showing significant difference between normal individual and cancer-developed individual. For the purpose of the present disclosure, the biomarker is a NUPR1 isoform A, and its expression level is increased in cancer-developed individuals compared to normal individuals, and the lower the expression level, the better the prognosis.

As another aspect for achieving the above object, the present disclosure provides a composition for diagnosing or predicting prognosis of cancer, comprising an agent for measuring an expression or activity level of a NUPR1 isoform A.

In one embodiment of the present disclosure, the agent may be a primer set or probe that specifically binds to NUPR1 isoform A.

The phrase "expression or activity level of a NUPR1 isoform A" as used herein means the mRNA expression level or protein activity level of the NUPR1 isoform A, and the measurement of "mRNA expression level of a NUPR1 isoform A" is a process of confirming the presence and expression level of a biomarker gene in a biological sample for the purpose of diagnosing or predicting prognosis of various cancers, and means measuring the amount of mRNA using a preparation used in a method for measuring the level of mRNA transcribed from a target gene. Measuring the "protein activity level of a NUPR1 isoform A" is a process of confirming the presence and expression level of marker proteins in biological samples for the purpose of diagnosing or predicting prognosis of various cancers, and as an example, antibodies that specifically binds to the NUPR1 isoform A protein may be used to confirm the amount of protein.

As another aspect for achieving the above object, the present disclosure provides a kit for diagnosing or predicting prognosis of cancer, comprising the composition for diagnosing or predicting prognosis of cancer.

The "kit" as used herein may be a kit comprising a composition comprising an agent capable of detecting or amplifying a marker, a reagent for the detection or amplification, and a restriction enzyme. Reagents for the amplification may comprise, but are not limited to, dNTPs, DNA polymerase and buffer. The dNTPs comprise dATP, dCTP, dGTP, and dTTP, and the DNA polymerase is a thermostable DNA polymerase, and commercially available polymerases such as Taq DNA polymerase and Tth DNA polymerase may be used. The restriction enzyme may be, but is not limited to, one or more of BfaI or BsaAI.

The kit of the present disclosure may further include a user guide describing optimal reaction performance conditions. The guide may be a printed matter explaining how to use the kit, for example, a method for preparing a PCR buffer, proposed reaction conditions, etc. The guide may include a brochure in the form of a pamphlet or leaflet, a label affixed to the kit, and instructions on the surface of the package containing the kit. In addition, the guide may include information disclosed or provided through an electronic medium such as the Internet.

The kit may further comprise a reagent for amplifying a NUPR1 isoform A, and the reagent may comprise DNA polymerase, dNTPs, and a buffer.

As another aspect for achieving the above object, the present disclosure provides a method for screening a substance for inhibiting cancer cell proliferation or metastasis, the method comprising:
treating a candidate substance to a cancer cell line expressing a NUPR1 isoform A; and
measuring an expression level of the NUPR1 isoform A in the cancer cell line.

In the above measurement, when the expression level of a NUPR1 isoform A is decreased compared to the control not treated with the candidate substance, the candidate substance may be determined as a substance for inhibiting proliferation or metastasis of cancer cells.

In one embodiment of the present disclosure, the expression level of the NUPR1 isoform A protein may be measured by any one selected from the group consisting of SDS-PAGE, immunofluorescence, enzyme-linked immunosorbent assay (ELISA), mass spectrometry, and protein chip, but the present disclosure is not limited thereto.

As another aspect for achieving the above object, the present disclosure provides a method to provide an information for proliferation and metastasis of cancer cell, the method comprising: measuring an expression level of mRNA or protein of a NUPR1 isoform A in a sample derived from a subject; and determining a subject whose expression level of mRNA or protein of the NUPR1 isoform A is higher than that of a control group as an individual at risk of proliferation or metastasis of cancer cell.

The terms "cancer" and "measurement of the expression level" as used herein are described above.

The "subject-derived sample" as used herein means a direct object to be separated from the subject and measure the expression level of the target gene or protein, and comprises sample such as a tissue, cell, whole blood, serum, plasma, saliva, sputum, cerebrospinal fluid, or urine, etc. For example, the sample as used herein may be a sample for diagnosing whether an individual suspected of developing cancer has developed, or a sample used to predict a clinical result after treatment from an individual who has undergone surgical or chemical treatment for cancer, that is. the possibility of recurrence and survival prognosis.

Another embodiment of the present disclosure relates to a method for screening a drug for overcoming or treating anticancer drug resistance or a drug for enhancing sensitivity to an anticancer drug.

The screening method according to an embodiment of the present disclosure may include: *in vitro* treating a candidate substance to cancer cells or cancer tissues expressing a NUPR1 isoform A protein or a gene encoding the same; and measuring the activity or expression level of the NUPR1 isoform A protein or the expression level of a gene encoding the protein in the cancer cells or cancer tissues after treatment with the candidate substance.

The "screening" as used herein means to select a substance having a specific target property from a candidate group consisting of various substances by a specific manipulation or evaluation method.

In the present disclosure, the cancer cells may be isolated from a target individual, preferably an individual resistant to or likely to be resistant to anticancer drugs, or may be engineered to overexpress a NUPR1 isoform A protein or a gene encoding the same, and more specifically, may be transfected by introducing a recombinant vector containing a gene encoding a NUPR1 isoform A protein into cancer cells.

The "vector" as used herein means a means for expressing a target gene in a host cell. The vector comprises elements for expression of a target gene, and may comprise a replication origin, a promoter, an operator, a transcription termination sequence(terminator), etc., and may further comprise enzyme sites (e.g., restriction enzyme sites) appropriate to incorporate into the genome of a host cell and/or selectable markers to confirm successful introduction into host cells and/or ribosome binding sites (RBS) for translation into proteins, IRES (Internal Ribosome Entry Site), etc. The vector may be engineered by a conventional genetic engineering method to have the above fusion polynucleotide (fusion promoter) as a promoter. The vector may further comprise transcription control sequences (e.g., enhancers, etc.) other than the promoter.

The recombinant vector as used herein may be a viral or non-viral vector. The viral vector may be, but is not limited to, an adenovirus vector, a retroviral vector including lentivirus, an adeno-associated virus vector, or a herpes simplex virus vector, etc. In addition, the non-viral vector may be, but is not limited to, a plasmid vector, a bacteriophage vector, a liposome, a bacterial artificial chromosome, or a yeast artificial chromosome, etc.

In the present disclosure, the target gene in the recombinant vector may be operably linked to the fusion polynucleotide. The term "operably linked" refers to a functional linkage between a gene expression control sequence and another nucleotide sequence. The gene expression control sequences may be "operably linked" to control the transcription and/or translation of other nucleotide sequences. In the recombinant vector, in order for the fusion polynucleotide to be operably linked to the target gene, the fusion polynucleotide may be linked to the 5' end of the target gene. The recombinant vector of the present disclosure may be used as an expression vector for a target protein capable of expressing the target protein with high efficiency in an appropriate host cell when the gene encoding the target protein to be expressed is operably linked.

The recombinant vector of the present disclosure may further comprise a transcription control sequence. The transcription control sequence may be, but is not limited to, one or more species selected from the group consisting of a transcription termination sequence such as a polyadenylation sequence (pA); replication origins such as f1 origin of replication, SV40 origin of replication, pMB 1 origin of replication, adeno origin of replication, AAV origin of replication, BBV origin of replication, etc.

In addition, in the present disclosure, the recombinant vector may further comprise a selectable marker. The selectable marker is a gene for confirming whether a recombinant vector has been successfully introduced into a host cell or for constructing a stable cell line, and for example, may be one or more selected from the group consisting of a drug resistance gene such as antibiotics, a metabolism-related gene, a gene amplification gene, etc.

In the present disclosure, delivery (introduction) of the recombinant vector into cancer cells may use a delivery method well known in the art. Examples of the delivery method may include, but are not limited to, microinjection, calcium phosphate precipitation, electroporation, sonoporation, magnetofection using a magnetic field, liposome-mediated transfection, gene bombardment, the use of dendrimers and inorganic nanoparticles, etc.

In the present disclosure, the candidate substance may be, but is not limited to, at least one selected from the group consisting of natural compounds, synthetic compounds, RNA, DNA, polypeptides, enzymes, proteins, ligands, antibodies, antigens, bacterial or fungal metabolites, and bioactive molecules.

In the present disclosure, examples of the agent for measuring the activity or expression level of above-mentioned protein may include, but are not particularly limited to, at least one selected from the group consisting of antibodies, oligopeptides, ligands, peptide nucleic acid (PNA) and aptamers.

In the present disclosure, examples of methods for measuring or comparing the activity or expression level of the protein may include, but are not limited to, protein chip analysis, immunoassay, ligand binding assay, Matrix Assisted Laser Desorption/Ionization Time of Flight Mass Spectrometry (MALDI-TOF) analysis, Sulface Enhanced Laser Desorption/Ionization Time of Flight Mass Spectrometry (SELDI-TOF) analysis, radioimmunoassay, radioimmunodiffusion method, Oukteroni immunodiffusion method, rocket immunoelectrophoresis, tissue immunostaining, complement fixation assay, two-dimensional electrophoretic assay, Liquid chromatography-mass spectrometry (LC-MS), liquid chromatography-Mass Spectrometry/Mass Spectrometry (LC-MS/MS), Western blotting or enzyme linked immunosorbent assay (ELISA), etc.

In the present disclosure, the agent for measuring an expression level of the gene encoding the protein may include at least one selected from the group consisting of primers, probes, and antisense nucleotides, that specifically bind to the gene.

Since the information of the protein according to the present disclosure or the gene encoding the protein is known, those skilled in the art will be able to easily design primers, probes or antisense nucleotides, that specifically bind to the gene encoding the protein based on this information.

In the present disclosure, in the process of confirming the presence and expression level of the gene, examples of analysis methods for measuring an expression level of the gene include, but are not limited to, reverse transcription polymerase reaction (RT-PCR), competitive reverse transcription polymerase reaction (Competitive RT-PCR), real-time reverse transcription polymerase reaction (Real-time RT-PCR), RNase protection assay (RPA), Northern blotting, or DNA chip.

In the present disclosure, when the activity or expression level of the NUPR1 isoform A protein measured in the cancer cells or cancer tissues after treatment with the candidate substance is reduced, or the expression level of the gene encoding the protein is reduced, the method may further include determining the candidate substance as a drug for overcoming or treating resistance to an anticancer drug or a drug for enhancing sensitivity to an anticancer drug.

In the screening method of the present disclosure, the description of the anticancer drug and cancer is the same as that described in the composition for diagnosing anticancer drug resistance.

As another embodiment for achieving the above object, the present disclosure provides a food composition for preventing or improving cancer, comprising a NUPR1 isoform A inhibitor as an active ingredient.

The terms NUPR1, NUPR1 isoform A inhibitor, and cancer as used herein are as described above.

The food composition of the present disclosure may be produced in various types of formulations, and unlike general drugs, has an advantage in that there is no side effect that may occur when taking a drug for a long-time using food as a raw material. The food composition according to the present disclosure may be produced in any form, and specifically, may be, but is not particularly limited to, one or more formulations selected from the group consisting of health functional food preparations such as tablets, capsules, pills, granules, liquids, powders, flakes, pastes, syrups, gels, jellies, bars, beverages, gums, and candies.

In addition, the food composition according to the present disclosure may include food additives in addition to the active ingredient. Food additives may generally be understood as substances that are added to, mixed with, or infiltrated into food in manufacturing, processing, or preserving food, and their safety should be guaranteed because they are consumed daily and for a long period of time together with food. The food additives are classified into sweeteners, flavoring agents, preservatives, emulsifiers, acidulants, thickeners, etc. in terms of function, and are not particularly limited as long as the food composition of the present disclosure meets the purpose to be achieved. In addition, the food composition according to the present disclosure may include, in addition to the above food additives, physiologically active substances or minerals that are known in the art for the purpose of functionality and nutritional supplementation and whose stability is guaranteed as food additives. The physiologically active substance or mineral is not particularly limited as long as it meets the purpose to be achieved by the food composition according to the present disclosure.

The food composition according to the present disclosure may comprise the above-mentioned food additives in an effective amount to achieve the purpose of addition according to the type of product, and in relation to other food additives that may be included in the food composition according to the present disclosure, reference may be made to the food code of each country or the Food Additives Codex.

As another embodiment for achieving the above object, the present disclosure provides a feed composition for preventing or improving cancer, comprising a NUPR1 isoform A inhibitor as an active ingredient.

The feed composition may comprise a feed additive. The feed additive according to the present disclosure corresponds to supplementary feed under the Feed Management Act.

The term "feed" as used herein may mean any natural or artificial diet, meal, etc., or components of the meal, especially for or suitable for eating, ingesting, and digesting by animals.

The type of feed is not particularly limited, and feeds commonly used in the art may be used. Non-limiting examples of the feed include vegetable feeds such as grains, root fruits, food processing by-products, algae, fibers, pharmaceutical by-products, oils and fats, starches, meal, or grain by-products; and animal feed such as proteins, inorganic materials, oils and fats, mineral oils, single cell proteins, zooplankton, or food. These may be used alone or in combination of two or more.

### [Advantageous Effects]

Since the NUPR1 isoform A inhibitor according to the present disclosure has an anticancer effect, pharmaceutical compositions, food compositions, and feed compositions comprising the NUPR1 isoform A inhibitor may be usefully used for preventing, treating, or improving cancer. In addition, by measuring an expression or activity level of the NUPR1 isoform A, it is possible to diagnose or predict the prognosis of cancer, substances for inhibiting cancer cell proliferation or metastasis may be screened, and information for cancer cell proliferation or metastasis may be provided to a subject.

### [Brief Description of the Drawings]

FIG. 1 is a result of Western blot analysis of the protein expression level of a NUPR1 isoform A in cancer cell lines.
FIG. 2 shows molecular docking simulation results of a NUPR1 isoform A and 3dc, and a NUPR1 isoform B and 3dc, respectively.
FIG. 3 is a result of measuring calorimetric titration of 3dc and a NUPR1 isoform A.
FIG. 4 shows molecular docking simulation results for a NUPR1 isoform A or a NUPR1 isoform B of 3dc, trifluoperazine (TFP) and ZZW-115.
FIG. 5 shows an expression level of a NUPR1 isoform A in GBM-28 and GBM-37 cell lines treated/untreated with 3dc, as the mRNA level.
FIG. 6 shows a result of immunochemical analysis wherein GBM-37 was treated with 3dc.
FIG. 7 shows cholecystokinin (CCK) results of GBM-28 and GBM-37 treated with temozolomide and 3dc, respectively.
FIG. 8 shows ae result of wound healing analysis of GBM-28 and GBM-37 treated with 3dc.
FIG. 9 shows invasion and proliferation inhibitory effects of GBM-37 treated with 3dc.
FIG. 10 shows changes in cell viability of GBM-28 and GBM-37 according to increasing treatment concentrations of 3dc.
FIG. 11 shows cell proliferation assay (MTS assay) results of GBM-28 and GBM-37 treated with 3dc and/or transfected with siRNAs of SEQ ID NOs: 5 and 6 (siNUPR1a) into a NUPR1 isoform A.
FIG. 12 shows a result of cDNAmicroarray analysis of GBM-37 treated with 3dc.
FIG. 13 is a result showing scatter plot and volcano plot of transcriptome changes of GBM-37 treated with 3dc.
FIG. 14 is a result of confirming the induction of transcriptome changes in GBM-37 treated with 3dc using a principal component analysis plot.
FIG. 15 is a result showing the gene expression regulation of GBM-37 treated with 3dc.
FIG. 16 shows a heatmap analysis result of GBM-37 treated with 3dc.
FIG. 17 shows gene ontology and network analysis related to biological processes of GBM-37 treated with 3dc.
FIG. 18 shows gene ontology and network analysis of cellular components in the gene of GBM-37 treated with 3dc.
FIG. 19 shows gene ontology and network analysis of gene molecular functions of GBM-37 treated with 3dc.
FIG.20 shows a result of genetic abnormality analysis of GBM-37 treated with 3 dc.
FIG.21 is a result of confirming the anticancer efficacy of 3dc in prostate cancer cells DU145 and PC3.
FIG.22 is a result of confirming the anticancer efficacy of 3dc in colon cancer cells, HT29 and HCT116.
FIG.23 is a result of confirming the anticancer efficacy of 3dc in a mouse model injected with colon cancer cells, HT29 and HCT116.
FIG.24 shows tumor appearance and H&E staining results at 4 weeks after 3dc treatment in a mouse model injected with colon cancer cells, HCT116.
FIG.25 is a result of confirming the anticancer efficacy of 3dc in thyroid cancer cells, SNU80 and SNU790.
FIG.26 is a result of confirming the anticancer efficacy of 3dc in lung cancer cells, A549 and NCI-H1299.
FIG.27 is a result of confirming the anticancer efficacy of 3dc in a mouse model injected with lung cancer cells, A549 and NCI-H1299.
FIG.28 is tumor appearance and H&E staining results at 4 weeks after 3dc treatment in a mouse model injected with lung cancer cells, A549.
FIG.29 is a result of confirming the anticancer efficacy of 3dc in skin cancer cells, A375P and SK-MEL1.
FIG.30 is a result of confirming the anticancer efficacy of 3dc in ovary cancer cells, Caov3 and SKOV3.

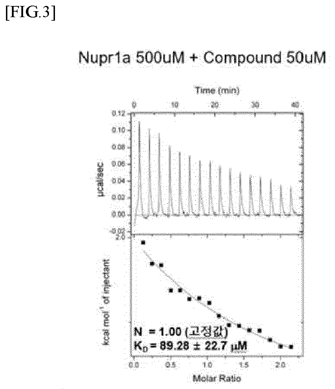

FIG.3 1 is a result of confirming the anticancer efficacy of 3dc in liver cancer cells, HepG2 and Hep3B.

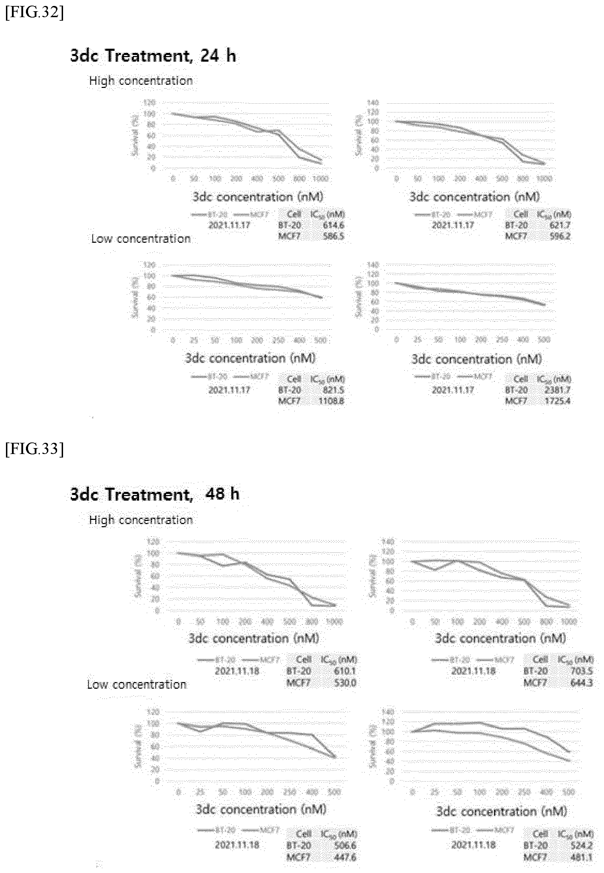

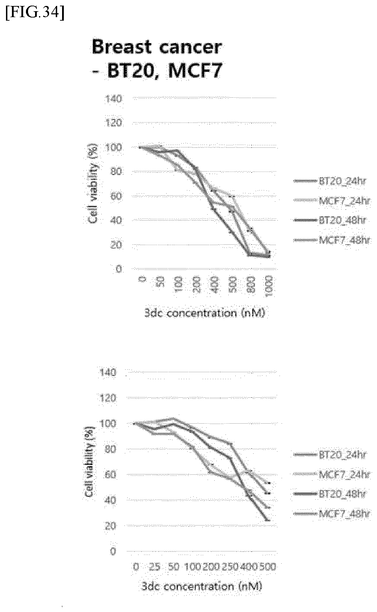

FIGS.32 to 34 are the results of confirming the anticancer efficacy of 3dc in breast cancer cells, BT-20 and MCF7.

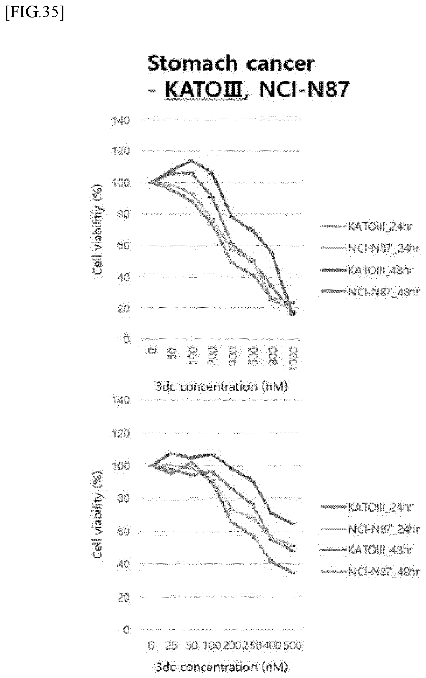

FIG.35 is a result of confirming the anticancer efficacy of 3dc in gastric cancer cells KATOIII and NCl-N87.

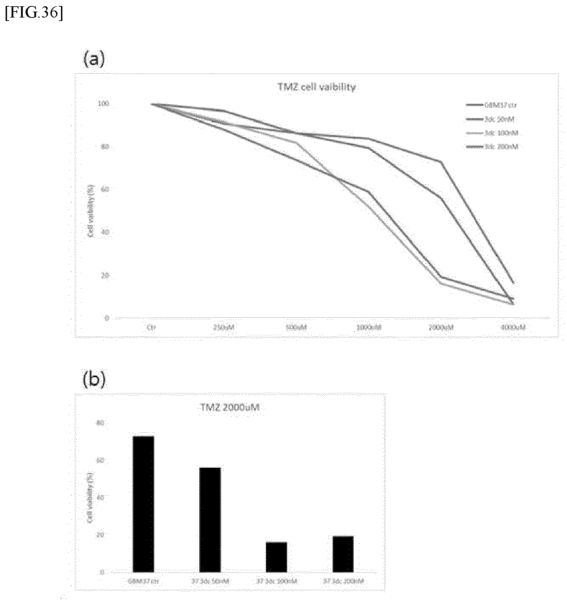

FIG.36 shows a result of confirming the sensitivity of 3dc to temozolomide by treating brain tumor cell GBM-37 with 3dc and temozolomide (a - confirming viability of GBM-37 depending on the concentration of 3dc and temozolomide, b - confirming cell viability by 3dc concentration each when treated with 2000 µM terozolomide).

### [Best Mode]

Hereinafter, the configuration and effects of the present disclosure will be described in more detail through examples. These examples are only for illustrating the present disclosure, and the scope of the present disclosure is not limited thereto.

### Example 1: Analysis of inhibition of NUPR1 isoform A expression by NUPR1 isoform A inhibitor

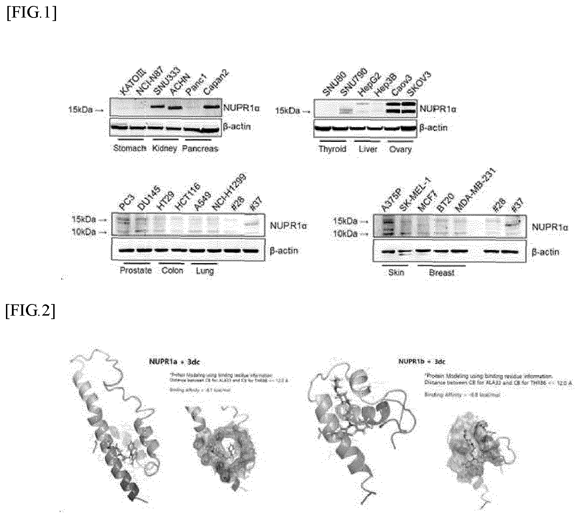

The protein expression level of a NUPR1 isoform A in various types of cancer cell lines including stomach, kidney, pancreas, thyroid, liver, ovary, prostate, colon, lung, skin, and breast was analyzed by Western blot. As a control, β-actin was used. As a result, it was confirmed that the NUPR1 isoform A was expressed in all types of cancer cell lines (FIG. 1).

Then, 10-(4-(4-(pyrrolidin-1-yl)piperidin-1-yl)butyl)-2-(trifluoromethyl)-10H-phenothiazine (hereinafter, referred to as 3dc) of Formular 1 below was selected as a candidate inhibitor of the NUPR1 isoform A:

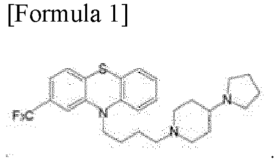

In order to confirm whether 3dc may have NUPR1 isoform A inhibitory efficacy, molecular docking simulations of the NUPR1 isoform A and 3dc, the NUPR1 isoform B and 3dc, respectively, were performed. As a result, as shown in FIG.2, the NUPR1 isoform A was predicted to show stronger binding to 3dc than the NUPR1 isoform B.

Additionally, calorimetric titrations of 3dc and the NUPR1 isoform A were determined. As a result, as shown in FIG.3, the binding affinity between the NUPR1 isoform A and 3dc was confirmed to be 89 uM, and this result shows that the binding of 3dc and the NUPR1 isoform A generates endothermic heat and structural changes in NUPR1 isoform A indicates the occurrence of structural change.

In addition, molecular docking simulations were performed for the NUPR1 isoform A or the NUPR1 isoform B of 3dc, trifluoperazine (TFP), and ZZW-115.

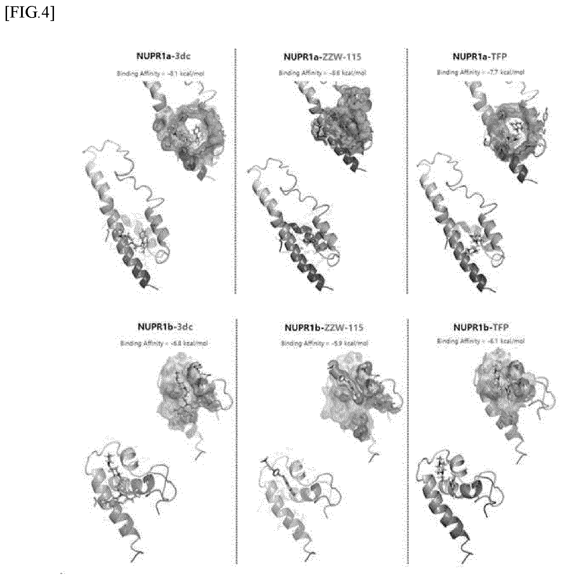

As a result, it was confirmed that all of 3dc, TFP, and ZZW-115 showed stronger binding to the NUPR1 isoform A than the NUPR1 isoform B (FIG.4), and these results suggest that NUPR1 isoform A plays a more important role in cell proliferation than NUPR1 isoform B.

### Experimental example 1: Confirmation of anti-cancer efficacy of NUPR1 isoform A inhibitor

### 1-1. Analysis of anticancer efficacy in brain tumor

In order to confirm the anticancer efficacy of NUPR1 isoform A inhibitors in brain tumors, experiments were performed using brain tumor patient-derived cell lines obtained from primary glioblastoma patients (GBM-28) and secondary gliosarcoma patients (GBM-37).

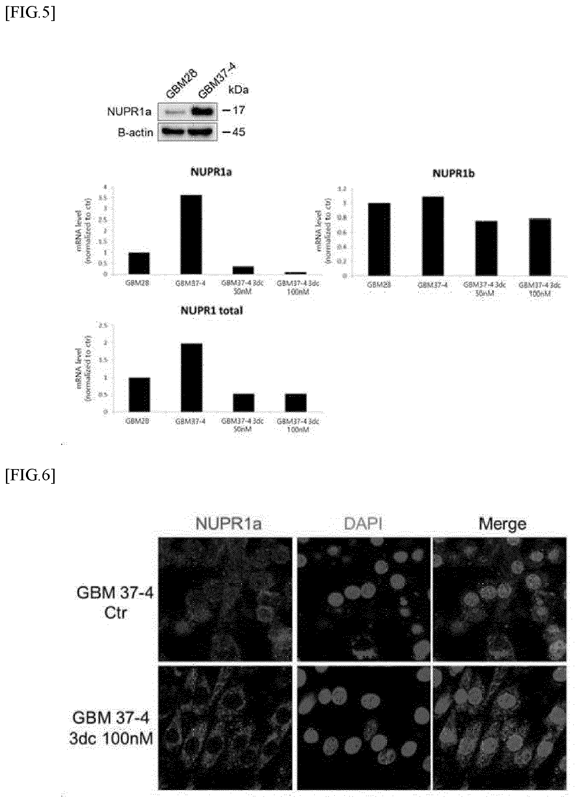

As shown in FIG.5, it was confirmed that GBM-37, which has a worse prognosis, expressed NUPR1 isoform A at a much higher level than GBM-28, and the mRNA expression level significantly decreased when treated with 3dc.

First, through immunochemical analysis, it was confirmed that when GBM-37 was treated with 3dc, nuclear transfer of the NUPR1 isoform A was inhibited, as shown in FIG.6.

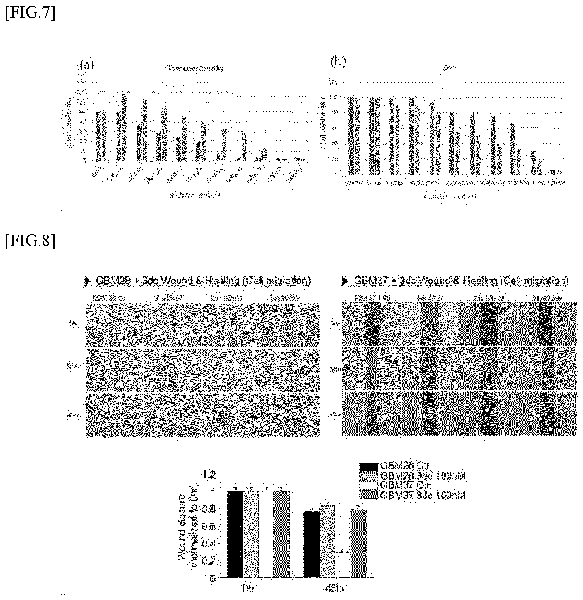

In addition, after treating GBM-28 and GBM-37 with temozolomide and 3dc, respectively, cholecystokinin (CCK) results were measured. As a result, as shown in FIG.7, it was confirmed that GBM-37, which expresses the NUPR1 isoform A at a higher level than GBM-28, showed a better effect on 3dc treatment.

Additionally, as a result of performing a wound healing assay after treating GBM-28 and GBM-37 with 3dc, it was confirmed that as shown in FIG.8, when GBM-37 expressing the NUPR1 isoform A at a higher level than GBM-28 was treated with 3dc, it had a better inhibitory effect on cell migration.

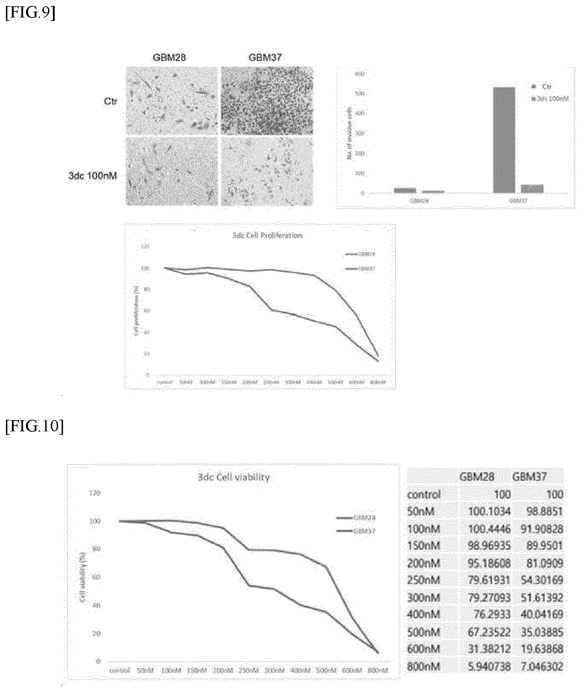

In addition, as a result of invasion and proliferation assay after treatment of GBM-28 and GBM-37 with 3dc, it was confirmed that as shown in FIG.9, when GBM-37 was treated with 3dc, invasion and proliferation inhibitory effects was better, and as shown in Fig 10, the cell viability of GBM-28 and GBM-37 decreased as the treatment concentration of 3dc increased.

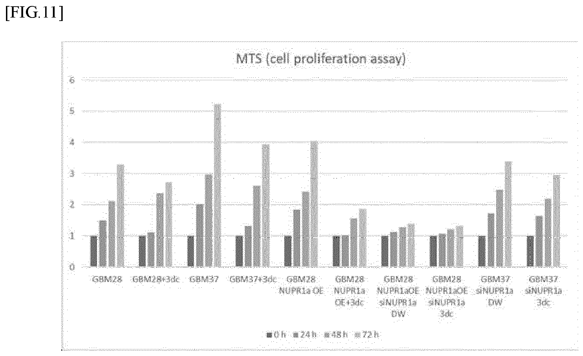

In addition, after treating GBM-28 and GBM-37 with 3dc, cell proliferation analysis (MTS assay) was performed. As a result of treating GBM-28 and GBM-37 with 3dc, respectively, it was confirmed that cell proliferation was inhibited compared to the untreated control group, and compared to the control group overexpressing (OE) NUPR1 isoform A in GBM-28, in the case of i) treating 3dc, ii) transfecting the NUPR1 isoform A with siRNAs of SEQ ID NOs: 5 and 6 (siNUPR1a), and iii) transfecting 3dc-treated NUPR1 isoform A with siRNAs of SEQ ID NOs: 5 and 6, the cell proliferation was all inhibited. In addition, compared to the untreated control GBM-37, in the case of i) transfecting the NUPR1 isoform A with siRNAs of SEQ ID NOs: 5 and 6 and ii) transfecting 3dc-trated NUPR1 isoform A with siRNAs of SEQ ID NOs: 5 and 6, the cell proliferation was all inhibited, confirming that 3dc inhibited the proliferation ability of brain tumor cells expressing the NUPR1 isoform A (FIG. 11).

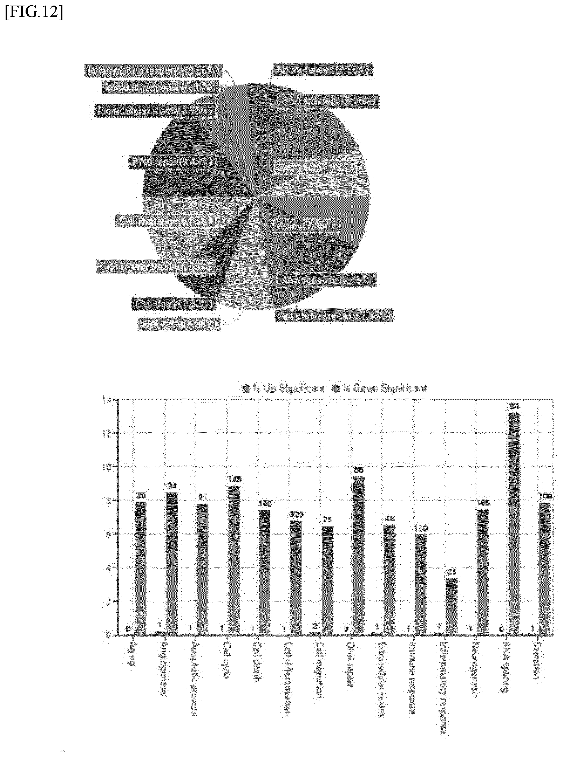

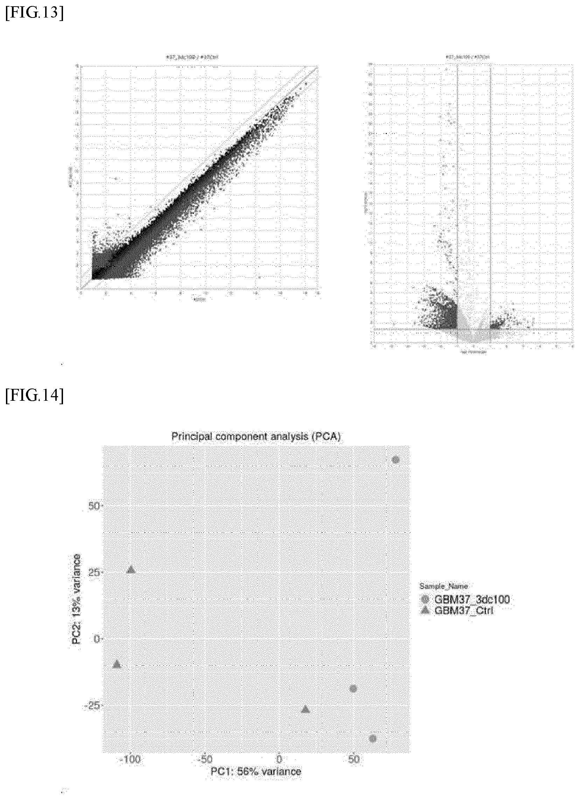

In addition, GBM-37 was treated with 100 nM of 3dc, and transcriptome changes were confirmed through cDNA microarray analysis. As a result, when 3dc was added (experimental group) compared to the control group (GBM-37), it was confirmed that genes related to essential cell phenomena such as DNA repair, cell migration, and cell cycle, etc. were down-regulated (FIG. 12), suggesting that 3dc treatment disrupted brain tumor cell function. As a result of representing the difference in gene expression between the control group and the experimental group as a scatter plot and a volcano plot (FIG. 13), it was confirmed that the experimental group treated with 3dc in both the scatter plot (left) and the volcano plot (right) showed that the number of down-regulated gene (green) of GBM-37 was significantly greater than the number of up-regulated genes (red).

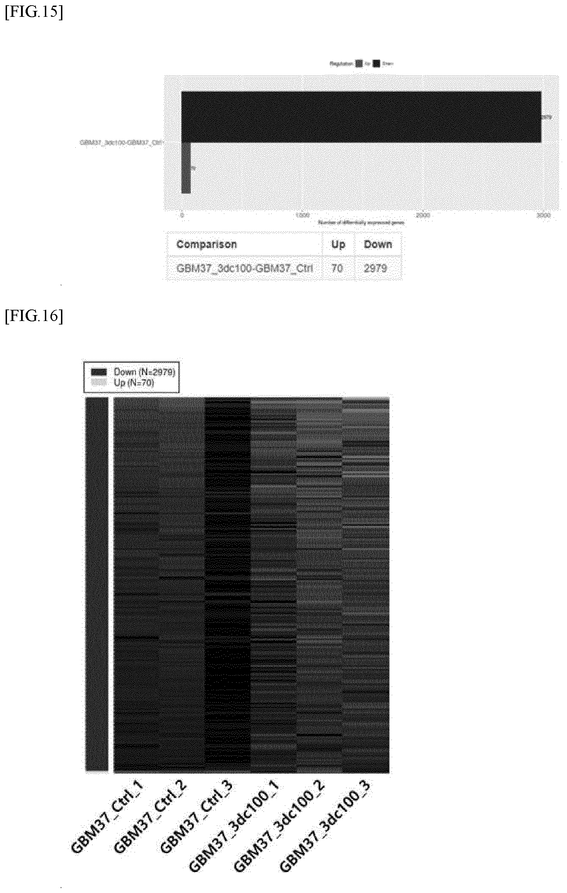

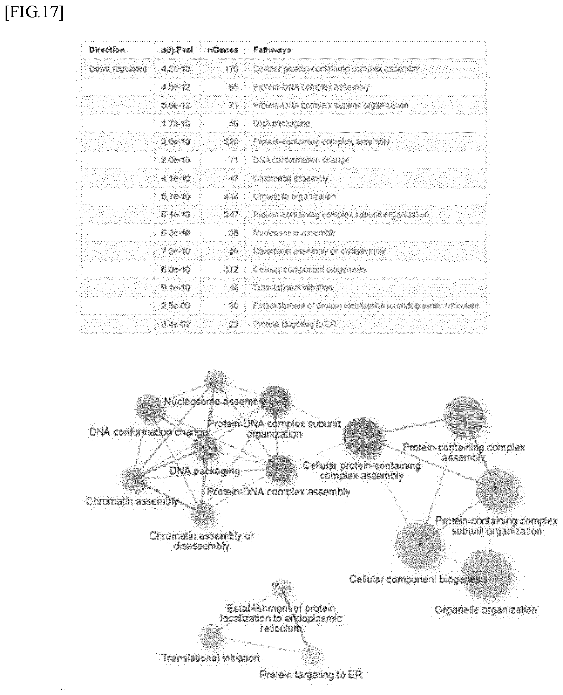

In addition, it was confirmed that when 3dc was added (experimental group) compared to the control group (GBM-37), it appeared to share transcriptional characteristics similar to the control group through the principal component analysis plot, and the overall transcriptome changes of the GBM-37 cell line by 3dc treatment were induced (FIG. 14), and 2979 genes in the experimental group were down-regulated (blue) compared to the control group, whereas only 70 genes were up-regulated (red) (FIG. 15). Similarly, it was confirmed that similarly in the heatmap analysis, when 3dc was added (experimental group) compared to the control group (GBM-37), 2979 genes were down-regulated (green) in the experimental group compared to the control group, whereas only 70 genes were up-regulated (red) (FIG. 16). The upper part of FIG. 17 shows the biological process-related gene ontology based on the transcriptome analysis, and represents biological processes enriched in down-stream genes when 3dc is added compared to the control group (GBM-37). The lower part of FIG. 17 shows the network analysis between the pathways listed in the upper table, and the darker the circle, the richer the gene set, the larger the circle, the larger the gene set, and the thicker the edge, the more overlapping genes. As a result of this analysis, it was confirmed that basic biological processes such as DNA packaging and protein-DNA complex assembly, etc., were significantly inhibited when brain tumor cells were treated with 3dc.

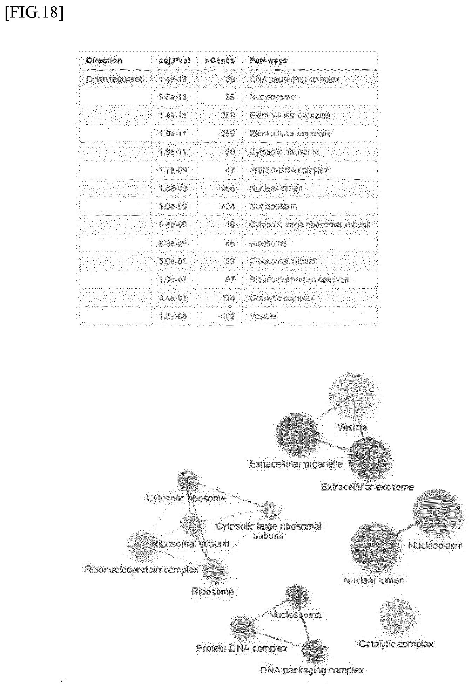

The upper part of FIG. 18 shows the gene ontology for cellular components within genes based on the transcriptome analysis, and shows cellular components enriched in genes that are down-regulated when 3dc is added compared to the control group (GBM-37). The lower part of FIG. 18 shows the network analysis between the pathways listed in the upper table, showing that basic cellular components such as DNA packaging complexes and nucleosomes are destroyed when brain tumor cells are treated with 3dc.

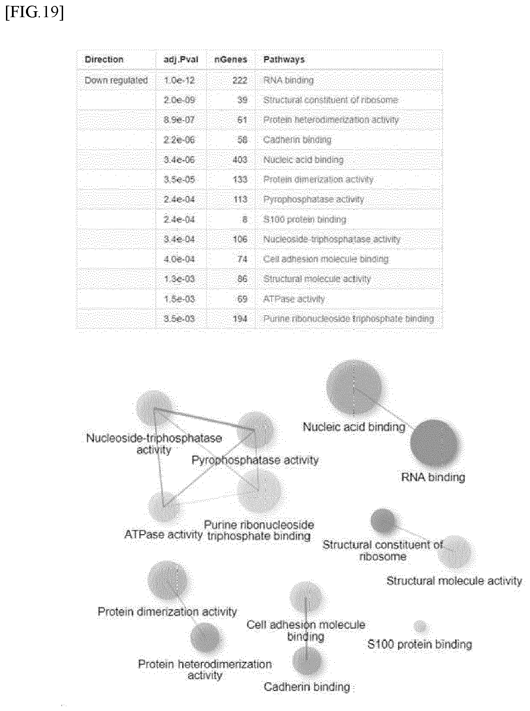

The upper part of FIG. 19 shows the gene ontology for the function of gene molecule based on the transcriptome analysis, and shows function of gene molecule enriched in genes that are down-regulated when 3dc is added compared to the control group (GBM-37). The lower part of FIG. 19 shows a network analysis between the pathways listed in the top table, showing that basic molecular functions such as nucleic acid binding and RNA binding are significantly inhibited when brain tumor cells are treated with 3dc.

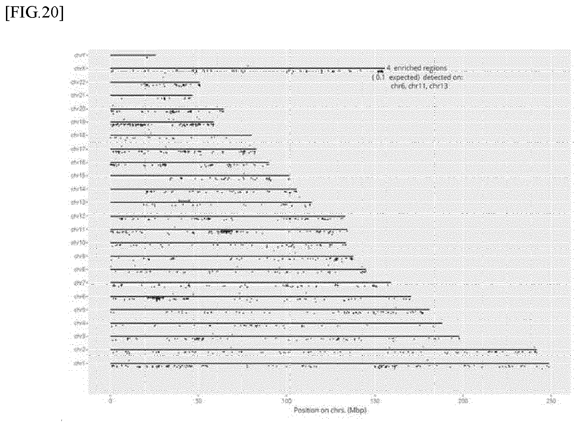

Based on the transcriptome analysis, genomic aberrations of coding genes that were down-regulated when 3dc was added compared to the control group were analyzed, and as a result, it was confirmed that most chromosomal genes were affected or down-regulated (blue) when treated with 3dc (FIG.20).

### 1-2. Analysis of anticancer efficacy in prostate cancer

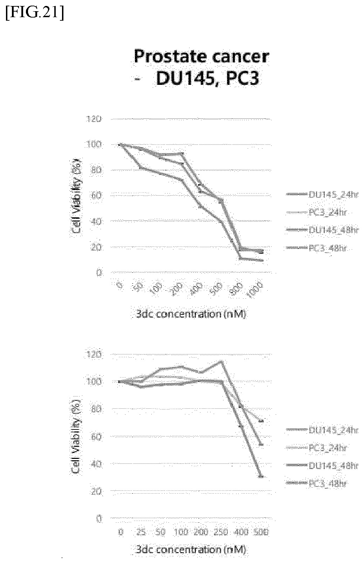

For prostate cancer cells, DU145 and PC3, as the treatment concentration of 3dc, a NUPR1 isoform A inhibitor, increased, cell viability decreased, confirming the anticancer efficacy of 3dc (FIG.21).

### 1-3. Analysis of anticancer efficacy in colon cancer

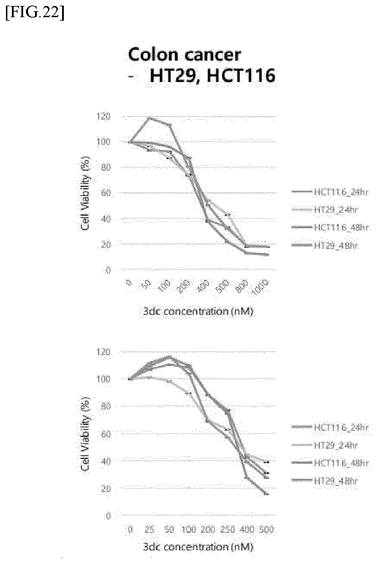

For colon cancer cells, HT29 and HCT116, as the treatment concentration of 3dc, a NUPR1 isoform A inhibitor, increased, cell viability decreased, confirming the anticancer efficacy of 3dc (FIG.22).

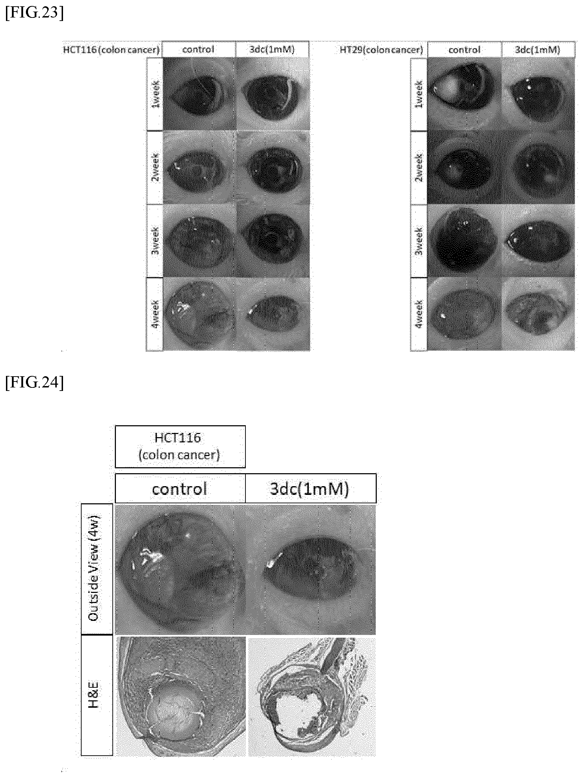

In addition, colon cancer cells, HT29 and HCT116 was intravitreal injected into 6-week-old male Balb/c nude mice at 3 × 10⁵ cells/2 µl according to a method disclosed in Lee et al. Experimental & Molecular Medicine (2019) 51:43, and then the tumor size of 1 mM 3dc treatment group (experimental group) and the untreated group (control group) was visually observed after 1 week, 2 weeks, 3 weeks and 4 weeks. As a result, it was confirmed that the tumor size of the 3dc-treated group was smaller and more transparent than that of the control group, confirming the anticancer efficacy of 3dc (FIG.23). FIG.24 shows tumor pictures and H&E staining results of the 3dc-treated group and the control group in HCT116 cells at 4 weeks.

### 1-4. Analysis of anticancer efficacy in thyroid cancer

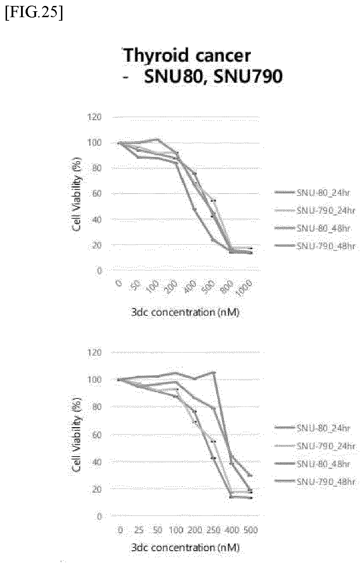

For thyroid cancer cells, SNU80 and SNU790, as the treatment concentration of 3dc, a NUPR1 isoform A inhibitor, increased, cell viability decreased, confirming the anticancer efficacy of 3dc (FIG.25).

### 1-5. Analysis of anticancer efficacy in lung cancer

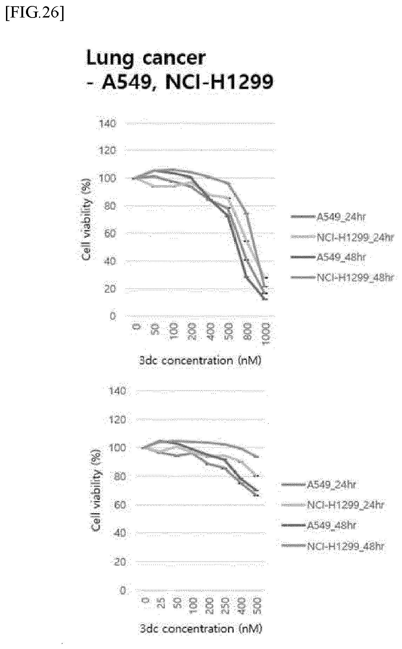

For lung cancer cells, A549 and NCI-H1299, as the treatment concentration of 3dc, a NUPR1 isoform A inhibitor, increased, cell viability decreased, confirming the anticancer efficacy of 3dc (FIG.26).

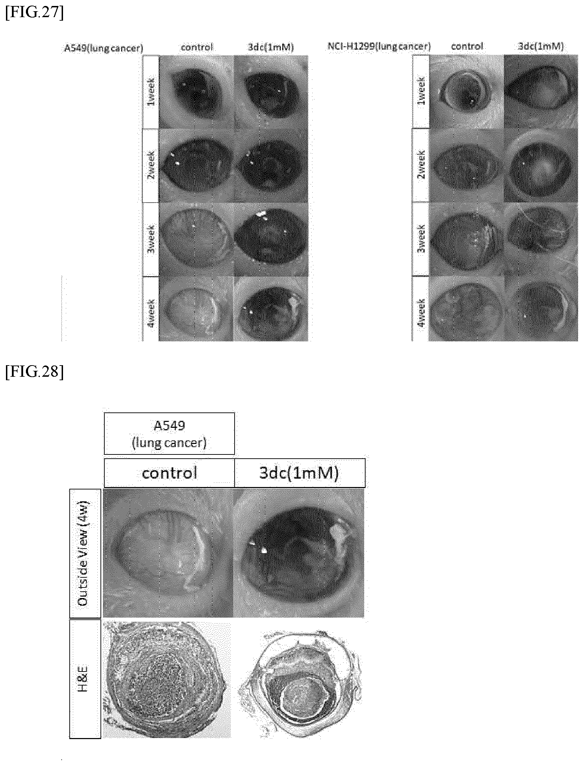

In addition, lung cancer cells, A549 and NCI-H1299 was intravitreal injected into 6-week-old male Balb/c nude mice at 3 × 10⁵ cells/2 µl according to a method disclosed in Lee et al. Experimental & Molecular Medicine (2019) 51:43, and then the tumor size of 1 mM 3dc treatment group (experimental group) and the untreated group (control group) was visually observed after 1 week, 2 weeks, 3 weeks and 4 weeks. As a result, it was confirmed that the tumor size of the 3dc-treated group was smaller and more transparent than that of the control group, confirming the anticancer efficacy of 3dc (FIG.27). FIG.28 shows tumor pictures and H&E staining results of the 3dc-treated group and the control group in A549 cells at 4 weeks.

### 1-6. Analysis of anticancer efficacy in skin cancer

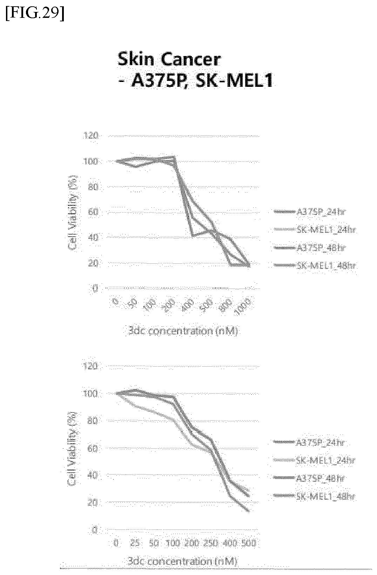

For skin cancer cells, A375P and SK-MEL1, as the treatment concentration of 3dc, a NUPR1 isoform A inhibitor, increased, cell viability decreased, confirming the anticancer efficacy of 3dc (FIG.29).

### 1-7. Analysis of anticancer efficacy in ovary cancer

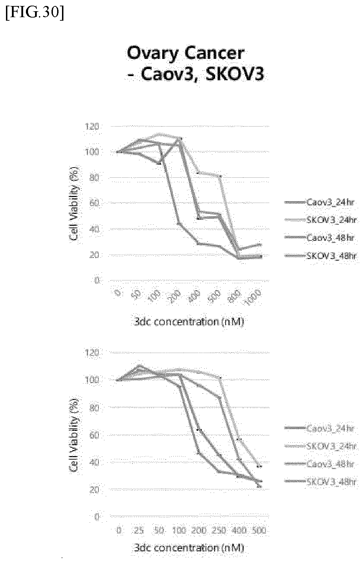

For ovary cancer cells, Caov3 and SKOV3, as the treatment concentration of 3dc, a NUPR1 isoform A inhibitor, increased, cell viability decreased, confirming the anticancer efficacy of 3dc (FIG.30).

### 1-8. Analysis of anticancer efficacy in liver cancer

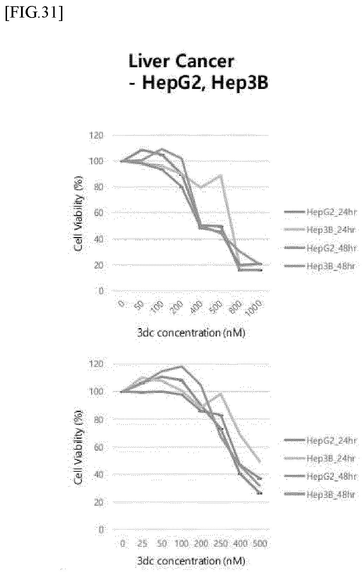

For liver cancer cells, HepG2 and Hep3B, as the treatment concentration of 3dc, a NUPR1 isoform A inhibitor, increased, cell viability decreased, confirming the anticancer efficacy of 3dc (FIG.31).

### 1-9. Analysis of anticancer efficacy in breast cancer

For breast cancer cells, BT-20 and MCF7, as the treatment concentration of 3dc, a NUPR1 isoform A inhibitor, increased, cell viability decreased, confirming the anticancer efficacy of 3dc (FIGS. 32, 33, and 34).

### 1-10. Analysis of anticancer efficacy in gastric cancer

For gastric cancer cells, KATOIII and NCl-N87, as the treatment concentration of 3dc, a NUPR1 isoform A inhibitor, increased, cell viability decreased, confirming the anticancer efficacy of 3dc (FIG.35).

### Experimental example 2: Confirmation of efficacy as anticancer adjuvant of NUPR1 isoform A inhibitor

To confirm whether 3dc, a NUPR1 isoform A inhibitor, may be applied as an anticancer adjuvant, brain tumor cell GBM-37 was treated with temozolomide (TMZ) and 3dc, and cell viability was confirmed.

Temozolomide is an FDA-approved alkylating agent and is the most widely used chemotherapy drug as a standard treatment regimen for brain tumor patients. The GBM-37 cell line is known to be more resistant to temozolomide than the GBM-28 cell line.

GBM-37 was pre-treated with 50 nM, 100 nM, and 200 nM of 3dc for 24 hours, and then was treated with 250 µM, 500 µM, 1000 µM, 2000 µM, and 4000 µM of temozolomide, respectively, and cell viability was confirmed.

As a result, in the case of GBM-37 pre-treated with 3dc, the cell viability was significantly reduced compared to the untreated control group, confirming that it was more sensitive to temozolomide (FIG.36A).

In addition, it was confirmed that when treated with 2000 µM of temozolomide, the cell viability of cell lines treated with 3dc at different concentrations (50 nM, 100 nM, and 200 nM) was significantly reduced compared to the untreated control group, so 3dc may be applied as an anticancer adjuvant by increasing the sensitivity of temozolomide in temozolomide-resistant GBM cell lines (FIG.36B).

From the above description, those skilled in the art to which the present disclosure pertains will be able to understand that the present disclosure may be embodied in other specific forms without changing the technical spirit or essential characteristics thereof. In this regard, it should be understood that the examples described above are illustrative in all respects and not limiting. The scope of the present disclosure should be construed as including all changes or modifications derived from the meaning and scope of the claims to be described later and equivalent concepts rather than the detailed description above are included in the scope of the present disclosure.

## Claims

1. A composition comprising a NUPR1 isoform A inhibitor.

2. The composition according to claim 1, wherein the NUPR1 isoform A inhibitor is any one selected from the group consisting of 10-(4-(4-phenylpiperazin-1-yl)butyl)-2-(trifluoromethyl)-10H-phenothiazine; 10-(4-(4-(2-fluorophenyl)piperazin-1-yl)butyl)-2-(trifluoromethyl)-10H-phenothiazine; 10-(3-(4-phenylpiperazin-1-yl)propyl)-2-(trifluoromethyl)-10H-phenothiazine; 10-(3-(4-(4-chlorobenzyl)piperazin-1-yl)propyl)-2-(trifluoromethyl)-10H-phenothiazine; 10-(3-(4-(4-) methoxyphenyl)piperazin-1-yl)propyl)-2-(trifluoromethyl)-10H-phenothiazine; 10-(3-(4-(4-nitrophenyl)piperazin-1-yl)propyl)-2-(trifluoromethyl)-10H-phenothiazine; 10-(3-(4-(3-chlorobenzyl)piperazin-1-yl)propyl)-2-(trifluoromethyl)-10H-phenothiazine; 10-(3-(4-(4-chlorophenyl)piperazin-1-yl)propyl)-2-(trifluoromethyl)-10H-phenothiazine; 10-(3-(4-(4-fluorobenzyl)piperazin-1-yl)propyl)-2-(trifluoromethyl)-10H-phenothiazine; 10-(3-(4-(2-chlorophenyl)piperazin-1-yl)propyl)-2-(trifluoromethyl)-10H-phenothiazine; 10-(3-(4-p-tolylpiperazin-1-yl)propyl)-2-(trifluoromethyl)-10H-phenothiazine; 10-(3-(4-phenethylpiperazin-1-yl)propyl)-2-(trifluoromethyl)-10H-phenothiazine; 10-(4-(4-(4-nitrophenyl)piperazin-1-yl)butyl)-2-(trifluoromethyl)-10H-phenothiazine; 10-(4-(4-(4-chlorophenyl)piperazin-1-yl)butyl)-2-(trifluoromethyl)-10H-phenothiazine; 2-chloro-10-(4-(4-phenylpiperazin-1-yl)butyl)-10H-phenothiazine; 2-chloro-10-(4-(4-(4-nitrophenyl)piperazin-1-yl)butyl)-10H-phenothiazine; 2-chloro-10-(4-(4-(4-methoxyphenyl)piperazin-1-yl)butyl)-10H-phenothiazine; 2-chloro-10-(4-(4-(4-chlorophenyl)piperazin-1-yl)butyl)-10H-phenothiazine; 10-(4-(4-(4-methoxyphenyl)piperazin-1-yl)butyl)-2-(trifluoromethyl)-10H-phenothiazine; 2-chloro-10-(3-(4-(4-methoxyphenyl)piperazin-1-yl)propyl)-10H-phenothiazine; 2-chloro-10-(3-(4-phenylpiperazin-1-yl)propyl)-10H-phenothiazine; 2-chloro-10-(3-(4-(4-nitrophenyl)piperazin-1-yl)propyl)-10H-phenothiazine; 2-chloro-10-(3 -(4-(4-chlorophenyl)piperazin-1-yl)propyl)-10H-phenothiazine; 10-(4-(4-phenylpiperidin-1-yl)butyl)-2-(trifluoromethyl)-10H-phenothiazine; 10-(4-(4-(4-methoxyphenyl)piperazin-1-yl)butyl)-2-(methylthio)-10H-phenothiazine; 2-(methylthio)-10-(4-(4-phenylpiperazin-1-yl)butyl)-10H-phenothiazine; 10-(3 -(4-phenylpiperidin-1 -yl)propyl)-2-(trifluoromethyl)-10H-phenothiazine; 2-(methylthio)-10-(4-(4-(4-nitrophenyl)piperazin-1-yl)butyl)-10H-phenothiazine; 10-(4-(4-(4-chlorophenyl)piperazin-1-yl)butyl)-2-(methylthio)-10H-phenothiazine; 2-(methylthio)-10-(3-(4-phenylpiperazin-1-yl)propyl)-10H-phenothiazine; 10-(3-(4-(4-chlorophenyl)piperazin-1-yl)propyl)-2-(methylthio)-10H-phenothiazine; 2-(methylthio)-10-(3-(4-(4-nitrophenyl)piperazin-1-yl)propyl)-10H-phenothiazine; 10-(3-(4-(4-methoxyphenyl)piperazin-1-yl)propyl)-2-(methylthio)-10H-phenothiazine; 2-chloro-10-(4-(4-phenylpiperidin-1-yl)butyl)-10H-phenothiazine; 2-chloro-10-(3-(4-phenylpiperidin-1-yl)propyl)-10H-phenothiazine; 2-(methylthio)-10-(3-(4-phenylpiperidin-1-yl)propyl)-10H-phenothiazine; 2-(methylthio)-10-(4-(4-phenylpiperidin-1-yl)butyl)-10H-phenothiazine; 10-(3-(1H-benzo[d]imidazol-1-yl)propyl)-2-(trifluoromethyl)-10H-phenothiazine; 2-(trifluoromethyl)-10-(3-(2,5,6-trimethyl-1H-benzo[d]imidazol-1-yl)propyl)-10H-phenothiazine; 2-(1-(4-(2-(trifluoromethyl)-10H-phenothiazine;-10-yl)butyl)piperidin-4-yl)ethanol; 4-(1-(4-(2-(trifluoromethyl)-10H-phenothiazine;-10-yl)butyl)piperidin-4-yl)morpholine; 10-(4-(4-(pyrrolidin-1-yl)piperidin-1-yl)butyl)-2-(trifluoromethyl)-10H-phenothiazine; 10-(4-(1,4'-bipiperidin-1'-yl)butyl)-2-(trifluoromethyl)-10H-phenothiazine; 4-(1-(4-(2-chloro-10H-phenothiazine;-10-yl)butyl)piperidin-4-yl)morpholine; 4-(1-(4-(2-(methylthio)-10H-phenothiazine;-10-yl)butyl)piperidin-4-yl)morpholine; 4-(1-(3-(2-(trifluoromethyl)-10H-phenothiazine;-10-yl)propyl)piperidin-4-yl)morpholine; 10-(4-(4,4-difluoropiperidin-1-yl)butyl)-2-(trifluoromethyl)-10H-phenothiazine; 10-(3-(4-(pyrrolidin-1-yl)piperidin-1-yl)propyl)-2-(trifluoromethyl)-10H-phenothiazine; 4-(1-(3-(2-chloro-10H-phenothiazine;-10-yl)propyl)piperidin-4-yl)morpholine; 2-chloro-10-(4-(4-(pyrrolidin-1-yl)piperidin-1-yl)butyl)-10H-phenothiazine; 2-chloro-10-(3-(4-(pyrrolidin-1-yl)piperidin-1-yl)propyl)-10H-phenothiazine; 2-(methylthio)-10-(4-(4-(pyrrolidin-1-yl)piperidin-1-yl)butyl)-10H-phenothiazine; 10-(3-(1,4'-bipiperidin-1'-yl)propyl)-2-(trifluoromethyl)-10H-phenothiazine; 4-(1-(3-(2-(methylthio)-10H-phenothiazine;-10-yl)propyl)piperidin-4-yl)morpholine; 2-(methylthio)-10-(3-(4-(pyrrolidin-1-yl)piperidin-1-yl)propyl)-10H-phenothiazine; 10-(4-(4,4-dimethylpiperidin-1-yl)butyl)-2-(trifluoromethyl)-10H-phenothiazine; 4-(1-(4-(10H-phenothiazine;-10-yl)butyl)piperidin-4-yl)morpholine; 10-(4-(4-(pyrrolidin-1-yl)p peridin-1-yl)butyl)-10H-phenothiazine; 10-(4-(1,4'-bipiperidin-1'-yl)butyl)-10H-phenothiazine; 2-methoxy-10-(4-(4-(pyrrolidin-1-yl)piperidin-1-yl)butyl)-10H-phenothiazine; 10-(4-(1,4'-bipiperidin-1'-yl)butyl)-2-methoxy-10H-phenothiazine; 4-(1-(4-(2-methoxy-10H-phenothiazine;-10-yl)butyl)piperidin-4-yl)morpholine; 10-(3-(4-(pyrrolidin-1-yl)piperidin-1-yl)propyl)-10H-phenothiazine; 10-(3-(1,4'-bipiperidin-1'-yl)propyl)-10H-phenothiazine; 4-(1-(3-(10H-phenothiazine;-10-yl)propyl)piperidin-4-yl)morpholine; 10-(3-(1,4'-bipiperidin-1'-yl)propyl)-2-methoxy-10H-phenothiazine; 4-(1-(3-(2-methoxy-10H-phenothiazine;-10-yl)propyl)piperidin-4-yl)morpholine; 2-methoxy-10-(3-(4-(pyrrolidin-1-yl)piperidin-1-yl)propyl)-10H-phenothiazine; 10-(3-(1,4'-bipiperidin-1'-yl)propyl)-2-chloro-10H-phenothiazine; 10-(4-(1,4'-bipiperidin-1'-yl)butyl)-2-chloro-10H-phenothiazine; 10-(3 -(1,4'-bipiperidin-1'-yl)propyl)-2-(methylthio)-10H-phenothiazine; 10-(4-(1,4'-bipiperin-1'-yl)butyl)-2-(methylthio)-10H-phenothiazine; 4-(1-(4-(2-fluoro-10H-phenothiazine;-10-yl)butyl)piperidin-4-yl)morpholine; 2-fluoro-10-(3-(4-methylpiperidin-1-yl)propyl)-10-phenothiazine; 4-(1-(3-(2-fluoro-10H-phenothiazine;-10-yl)butyl)piperidin-4-yl)morpholine; 2-fluoro-10-(4-(4-methylpiperidin-1-yl)butyl)-10H-phenothiazine; 2-fluoro-10-(4-(4-(pyrrolidin-1-yl)piperidin-1-yl)butyl)-10H-phenothiazine; 10-(4-(1,4 '-bipiperidin-1 '-yl)butyl)-2-fluoro-10H-phenothiazine; 4-(1-(3-(2-fluoro-10H-phenothiazine;-10-yl)propyl)piperidin-4-yl)morpholine; 10-(3-(1,4'-bipiperidin-1'-yl)propyl)-2-fluoro-10H-phenothiazine; 2-fluoro-10-(3-(4-(pyrrolidin-1-yl)piperidin-1-yl)propyl)-10H-phenothiazine; 3-methyl-10-(4-(4-(pyrrolidin-1-yl)piperidin-1-yl)butyl)-10H-phenothiazine; 10-(4-(1,4'-bipiperidin-1'-yl)butyl)-3-methyl-10H-phenothiazine; 4-(1-(4-(3-methyl-10H-phenothiazine;-10-yl)butyl)piperidin-4-yl)morpholine; 4-(1-(3-(3-methyl-10H-phenothiazine;-10-yl)propyl)piperidin-4-yl)morpholine; 10-(3-(1,4'-bipiperidin-1'-yl)propyl)-3-methyl-10H-phenothiazine; 3-methyl-10-(3-(4-(pyrrolidin-1-yl)piperidin-1-yl)propyl)-10H-phenothiazine; 10-(4-(4-morpholinopiperidin-1-yl)butyl)-10H-phenothiazine;-3-carbonitrile; 10-(4-(4-(pyrrolidin-1-yl)piperidin-1-yl)butyl)-1 0H-phenothiazine;-3-carbonitrile; 10-(4-(1,4'-bipiperidin-1'-yl)butyl)-10H-phenothiazine;-3-carbonitrile; 2-methyl-10-(4-(4-(pyrroli din-1-yl)pip eridin-1-yl)butyl)-10H-phenothiazine; and 10-(4-(1,4'-bipiperidin-1'-yl)butyl)-2-methyl-10H-phenothiazine.

3. The composition according to claim 1 or 2, wherein the NUPR1 isoform A inhibitor is 10-(4-(4-(pyrrolidin-1-yl)piperidin-1-yl)butyl)-2-trifluoromethyl)-10H-phenothiazine represented by Formula 1 or 2 or a derivative thereof,:

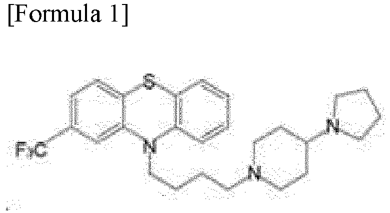

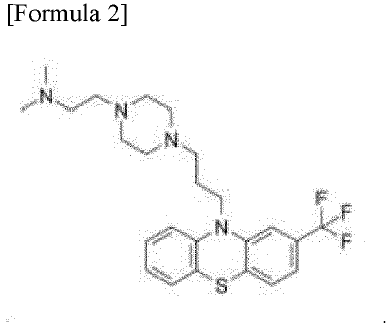

4. The composition according to any one of the preceding claims, wherein the composition is a pharmaceutical composition or a food composition.

5. The composition according to ant one of the preceding claims for use in preventing or treating cancer.

6. The composition for use according to claim 5, wherein the composition inhibits proliferation or metastasis of cancer cells by inhibiting NUPR1 isoform A gene expression or NUPR1 isoform A protein expression.

7. The composition for use according to claim 5 or 6, wherein the cancer is at least one selected from the group consisting of brain tumor, lung cancer, breast cancer, stomach cancer, large intestine cancer, kidney cancer, skin cancer, pancreatic cancer, liver cancer, bladder cancer, colon cancer, thyroid cancer, oral cancer, prostate cancer, rectal cancer, non-small cell lung cancer, labrum cancer, anal cancer, vulvar cancer, oropharyngeal cancer, nasopharyngeal cancer, urethral cancer, small intestine cancer, biliary tract cancer, ovarian cancer, laryngeal cancer, and esophageal cancer.

8. The composition for use according to any one claims 5 to 7, wherein the composition inhibits progression, malignancy, resistance, or metastasis of cancer.

9. NUPR1 isoform A inhibitor as defined in any one of claims 1 to 4 for use in treating anticancer drug resistance or enhancing anticancer drug sensitivity.

10. A composition for diagnosing or predicting prognosis of cancer, comprising an agent for measuring an expression or activity level of a NUPR1 isoform A,
preferably wherein the agent is a primer set or probe that specifically binds to the NUPR1 isoform A.

11. A kit for diagnosing or predicting prognosis of cancer, comprising the composition of claim 10,
preferably further comprising a reagent for amplifying the NUPR1 isoform A, more preferably wherein the reagent contains DNA polymerase, dNTPs, and buffer.

12. A method for screening a substance for inhibiting cancer cell proliferation or metastasis, comprising:
treating a candidate substance to a cancer cell line expressing a NUPR1 isoform A; and
measuring an expression level of the NUPR1 isoform A in the cancer cell line,
preferably wherein the cancer is at least one selected from the group consisting of brain tumor, lung cancer, breast cancer, stomach cancer, large intestine cancer, kidney cancer, skin cancer, pancreatic cancer, liver cancer, bladder cancer, colon cancer, thyroid cancer, oral cancer, prostate cancer, rectal cancer, non-small cell lung cancer, labrum cancer, anal cancer, vulvar cancer, oropharyngeal cancer, nasopharyngeal cancer, urethral cancer, small intestine cancer, biliary tract cancer, ovarian cancer, laryngeal cancer, and esophageal cancer.

13. The method of claim 12, wherein when the expression level of the NUPR1 isoform A is decreased compared to the control not treated with the candidate substance, the method includes determining the candidate substance as a substance for inhibiting proliferation or metastasis of cancer cells.

14. The method of claim 12 or 13, wherein the expression level of the NUPR1 isoform A protein is measured by any one selected from the group consisting of SDS-PAGE, immunofluorescence, enzyme-linked immunosorbent assay (ELISA), mass spectrometry, and protein chip.

15. A method of providing an information for cancer cell proliferation or metastasis, comprising:
measuring an expression level of mRNA or protein of a NUPR1 isoform A in a sample derived from a subject; and
determining the subject as an individual at risk of cancer cell proliferation or metastasis wherein the expression level of mRNA or protein of the NUPR1 isoform A in the subject is increased than a control group,
preferably wherein the cancer is at least one selected from the group consisting of brain tumor, lung cancer, breast cancer, stomach cancer, large intestine cancer, kidney cancer, skin cancer, pancreatic cancer, liver cancer, bladder cancer, colon cancer, thyroid cancer, oral cancer, prostate cancer, rectal cancer, non-small cell lung cancer, labrum cancer, anal cancer, vulvar cancer, oropharyngeal cancer, nasopharyngeal cancer, urethral cancer, small intestine cancer, biliary tract cancer, ovarian cancer, laryngeal cancer, and esophageal cancer.
